# EUROPÄISCHE PATENTSCHRIFT

(11) **EP 2 787 069 B1**
(45) Veröffentlichungstag und Bekanntmachung des Hinweises auf die Patenterteilung: **05.08.2015**
(21) Anmeldenummer: 13162391.0
(22) Anmeldetag: 04.04.2013
(51) Int. Cl.: C12M 1/26, C12M 1/42, C12M 1/12, A61L 27/36

(54) **Verfahren und Vorrichtung zur Dezellularisierung von Organen und Geweben**
Method and device for decellularising organs and tissues
Procédé et dispositif de décellularisation d'organes et de tissus

(43) Veröffentlichungstag der Anmeldung: 08.10.2014
(73) Patentinhaber: Universität Leipzig, 04109 Leipzig (DE)
(72) Erfinder: Koch, Holger, 04435 Schkeuditz (DE); Boldt, Andreas, 04177 Leipzig (DE); Sack, Ulrich, 04275 Leipzig (DE)
(74) Vertreter: Vossius & Partner Patentanwälte Rechtsanwälte mbB

(56) Entgegenhaltungen:
- EP-A1- 1 462 515
- WO-A1-01/82992
- WO-A2-2011/062621
- DE-A1- 10 021 627
- US-A1- 2003 087 428
- US-B1- 6 416 995

## Beschreibung

Die Erfindung betrifft ein Verfahren sowie eine Vorrichtung zur Dezellularisierung von Organen, Organsystemen und/oder Geweben. Insbesondere betrifft die Erfindung ein effektives und schonendes Verfahren zur Dezellularisierung, bei dem die Organe oder Gewebe laminaren und/oder turbulenten Strömungen und Verwirbelungen einer Reinigungsflüssigkeit ausgesetzt werden, sowie eine entsprechende Vorrichtung dafür. Einige Ausführungsformen der Erfindung erlauben zudem eine effektive Entfernung von an der Matrix gebundener Reinigungslösung.

Dezellularisierte Organe oder Gewebe werden unter anderem bei der Herstellung zellfreier Implantate zur Behandlung verschiedenster Krankheitsbilder benötigt. Beispielsweise kann ein zellfreies Speiseröhren-Implantat zur Behandlung von langstreckiger Ösophagusatresie bei Neugeborenen zum Einsatz kommen.

Die bisherige in der Literatur beschriebene Vorgehensweise zur Dezellularisierung von Geweben/Organen beinhaltet meist ein Perforieren oder Kanülieren des Gewebes/Organs.

Die US 2003/0087428 A1 beschreibt eine Vorrichtung und ein Verfahren zur Dezellularisierung von weichem Implantatgewebe mittels Behandlung durch eine Lösung auf anionischer Detergenzbasis durch Extraktionsverfahren mit anschließendem Waschvorgang mittels Wasser. Ein zu dezellularisierendes Gefäß wird auf eine Düse genäht und die Lösung hindurch geführt.

Aufgabe der Erfindung ist es, ein Verfahren sowie eine Vorrichtung zur Dezellularisierung von Organen, Organsystemen und/oder Geweben bereitzustellen, das ohne ein Perforieren oder Kanülieren der zu dezellularisierenden Organe/Gewebe auskommt. Eine weitere Aufgabe der vorliegenden Erfindung besteht darin, bei der Dezellularisierung von Organen/Geweben nur vergleichsweise geringe Mengen an Detergenzien (z.B. SDS) zur vollständigen Dezellularisierung zu benötigen. Ferner ist es Aufgabe der Erfindung, den Herstellungsprozess eines dezellularisierten Scaffolds zeitlich zu verkürzen, also die Effizienz der Herstellung zu verbessern.

Diese Aufgaben werden durch ein Verfahren sowie eine Vorrichtung mit den Merkmalen der vorliegenden Patentansprüche gelöst.

Insbesondere sind das erfindungsgemäße Verfahren und die erfindungsgemäße Vorrichtung für schlauchartige Hohlorgane wie Speiseröhre, Harnleiter, Harnröhre geeignet. Jedoch sind das erfindungsgemäße Verfahren und die erfindungsgemäße Vorrichtung unabhängig von der Art des zu dezellularisierenden Organs oder Gewebes, d.h. es können verschiedenste Gewebe, Organe und/oder Organsysteme dezellularisiert werden.

Der Begriff "Organ" umfasst sowohl ganze, isolierte Organe eines Tieres, aber auch einzelne, isolierte Bestandteile, wie beispielsweise Lungenflügel oder bronchiale Bestandteile und Strukturen. Der Begriff "Organ" umfasst weiterhin auch komplette Organsysteme, wie beispielsweise den Verdauungstrakt. Der Begriff "Organ" umfasst sowohl Säugetierorgane (auch menschliche) aber auch Organe von Vögeln und Reptilien, Insekten und Fischen.

Ein Organ, das in dem erfindungsgemäßen System verwendet wird, ist vorzugsweise entweder ein isoliertes Organ oder die Speiseröhre eines Ganztieres. Beispielsweise wird eine isolierte Speiseröhre eines Schweins verwendet. Der Fachmann wird jedoch ebenfalls andere tierische Organe, insbesondere tierische Speiseröhren und/oder Speiseröhrenbestandteile erfindungsgemäß einsetzen. Daher können auch andere isolierte Nager-, Insekten-, Vogel-, Reptilien- oder Säugetierorgane eingesetzt werden. Beispielsweise kann es sich um Organe wie Herz, Niere oder Leber größerer Säugetiere (z.B. vom Schwein, Schaf, Rind, Mensch), kleinerer Säuger (z.B. Maus, Ratte, Kaninchen) oder Organe anderer Tiertaxa wie etwa ein Krokodilherz oder Insektenorgane handeln. Organbestandteile im Sinne dieser Erfindung umfassen ganze Organe, allerdings auch Teile wie beispielsweise einzelne Lungenflügel einer Lunge oder bronchiale Bestandteile des Lungensystems. Ebenfalls können in bestimmten Situationen auch Zell- und Gewebekultursysteme eines Organs und/oder der Organbestandteile in den erfindungsgemäßem Systemen und Verfahren eingesetzt werden. Im Sinne der Erfindung können auch humane Organ- und/oder Organbestandteile (z.B. bronchiale Bestandteile einer Lunge) im erfindungsgemäßen System eingesetzt werden, wobei diese Organe nach ethischen Gesichtspunkten und Verfahren post mortem erhalten wurden. Bei experimentellen Verfahren (z.B. Durchmusterungsverfahren oder Verfahren zum Auffinden von geeigneten Applikationsmustern) werden bevorzuge isolierte Organe (oder deren Bestandteile) von Tieren (auch vom Menschen) eingesetzt.

Auf diese Weise können unterschiedliche Scaffolds zur Rezellularisierung bereitgestellt werden.

Ein Aspekt der Erfindung betrifft ein Verfahren gemäß Patentanspruch 1 zur Dezellularisierung von Organen oder Geweben in einem Reaktor. Dabei weist der Reaktor auf: mindestens eine erste, äußere Rohrdüse und mindestens eine zweite, innere Rohrdüsen, wobei die Rohrdüsen im Innenraum des Reaktors montiert sind und in die Rohrdüsen von außerhalb des Reaktors durch einen Zufluss Flüssigkeit zugeführt werden kann, und einen Abfluss. Jedes der zu dezellularisierenden Organe oder Gewebe ist dabei im Innenraum des Reaktors positioniert und auf eine zweite, innere Rohrdüse aufgezogen. Im Reaktor befindet sich eine Reinigungslösung. Das Verfahren weist dabei folgende Schritte auf:
(1a) Pressen einer Reinigungslösung mit Druck durch die jeweiligen Zuflüsse in die Rohrdüsen;
(1b) Ansaugen von Reinigungsflüssigkeit durch den Abfluss aus dem Reaktor.

Dabei ist der Druck in Schritt (1a) so gewählt, dass im Reaktor starke laminare und/oder turbulente Strömungen und Verwirbelungen in der Reinigungslösung auftreten, und die Schritte (1a) und (1b) werden vorzugsweise gleichzeitig und für die Dauer einer vorbestimmten Zeit ausgeführt.

Angaben über die Abmessungen (z.B. Höhe und Breite) sowie die Geometrie des Reaktors und der Rohrdüsen sind hier bewusst nicht angegeben, da diese an die Abmessungen des zu dezellularisierenden Organs oder Gewebes angepasst werden müssen. Werden Organe wie beispielsweise Herz, Niere oder Leber größerer Säugetiere (z.B. vom Schwein, Schaf, Rind, Mensch), kleinerer Säuger (z.B. Maus, Ratte, Kaninchen) oder Organe anderer Tiertaxa wie etwa ein Krokodilherz oder Insektenorgane dezellularisiert, macht dies eine entsprechende Anpassung der Geometrie des erfindungsgemäßen Reaktors auf das erforderliche Maß notwendig. Der Reaktor kann aus unterschiedlichsten, chemisch stabilen Materialien wie z.B. Glas, Acrylglas oder anderen Kunststoffen gefertigt werden.

Alle Rohrdüsen sind dabei so angebracht, dass sie gegebenenfalls wieder ausgebaut und gegen andere Rohrdüsen entsprechend der Organgröße (z.B. Speiseröhre Schwein vs. Speiseröhre Maus) ausgetauscht werden können, wobei sowohl der Durchmesser der Rohrdüsen als auch der Durchmesser der Austrittsöffnungen oder Düsen an der Rohrdüse variabel ist.

Ferner kann sich an der Oberseite des Reaktors eine Bohrung befinden, über welche ein System zur Befestigung der Organe (z.B. Netz, Haken) angebracht werden kann. Dieses Befestigungssystem ermöglicht das Aufhängen größerer oder filigraner Organe wie z.B. Niere, Herz, Magen oder Kapillarsystem.

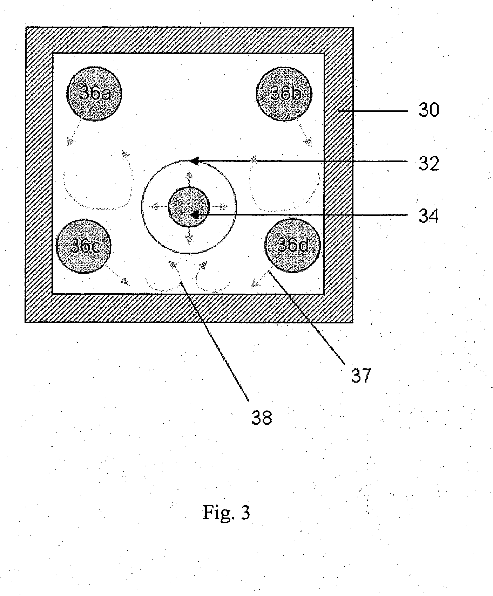

Größe und Material der Rohrdüsen (vgl. beispielsweise Figuren 3 - 5) sind variabel und können gemäß der Anforderungen ausgewählt werden, die sich beispielsweise aus den besonderen Eigenschaften (wie den Abmessungen) des zu dezellularisierenden Organs oder Gewebes oder aber auch bei der Herstellung des Reaktors ergeben. Der Innendurchmesser der Rohrdüsen liegt vorzugsweise im µm- bis cm-Bereich. Die Rohrdüsen können aus unterschiedlichsten, chemisch stabilen Materialien wie z.B. Glas, Acrylglas oder anderen Kunststoffen bestehen. In der Wand der Rohrdüsen befinden sich eine Anzahl von Austrittsöffnungen (im Folgenden auch einfach "Löcher" genannt), die beispielsweise durch Bohrungen realisiert werden können, oder Düsen. Die Anzahl sowie die Anordnung der Austrittsöffnungen oder Düsen kann dabei den Anforderungen (siehe oben) angepasst werden. Beispielsweise können Löcher in der Wand der Rohrdüse in Reihen angeordnet sein, die wiederum etwa parallel zu einer gegebenen Symmetrieachse der Rohrdüse verlaufen können. Die Anzahl der Löcher oder Düsen und/oder gegebenenfalls der Lochreihen können dabei den Bedürfnissen angepasst werden.

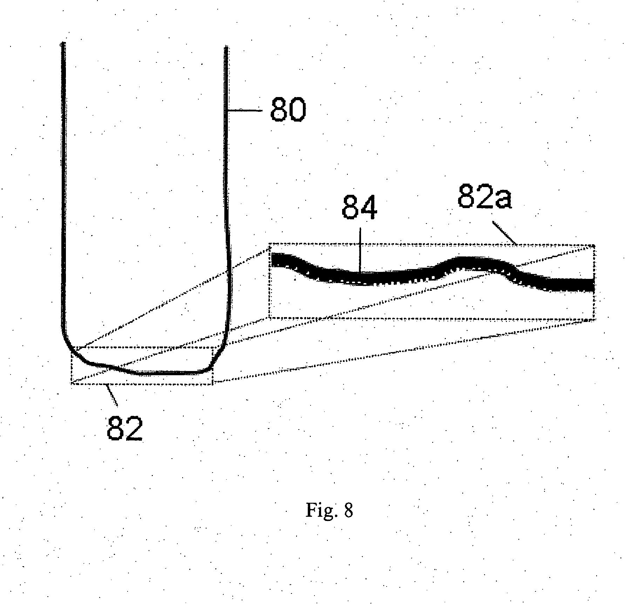

Der Begriff "Rohrdüsen" soll auch "Schlauchdüsen" umfassen. Unter "Schlauchdüsen" sollen Schläuche aus flexiblem, biegsamem Material verstanden werden, in deren Wand Austrittsöffnungen oder Düsen angebracht sind. Insbesondere sind Anzahl und Anordnung der Austrittsöffnungen oder Düsen dabei wie beschrieben, wenn die Schlauchdüse in eine gerade ausgedehnte Form gebracht wird. Vorzugsweise sind die Schlauchdüsen aus dünnen Kunststoffschläuchen gefertigt. Die flexiblen Schlauchdüsen können neben Rohrdüsen aus stabilem Material oder alternativ zu Rohrdüsen aus stabilem Material im Reaktor verwendet werden (vgl. beispielsweise Fig. 8). Bei den Schlauchdüsen wird vorzugsweise über beide Enden Flüssigkeit durch die Düsen gedrückt.

Somit können bei komplexen Organen (z.B. Herz oder Niere) Schlauchdüsen durch das gesamte Organlumen (linke Vorhof → linke Herzkammer → rechter Vorhof → rechte Herzkammer, oder anders herum) gezogen ("gefädelt") und somit das gesamte Organlumen mit Medium benetzt werden, ohne ein eigentliches "Kanülieren" des Organs vorzunehmen. Abhängig von der Größe der Spezies (z.B. Ratte oder Schwein) kommen hier Schlauchdüsen unterschiedlichen Innendurchmessers (vorzugsweise im µm- bis cm-Bereich) zum Einsatz.

Durch das Verwenden der Rohrdüsen entstehen bei ausreichend hohen Drücken in Schritt (1a) turbulente Strömungen der Reinigungslösung im Reaktionsgefäß, welche Kräfte auf die Oberfläche der Außen- (erste Rohrdüse(n), im Folgenden auch "äußere Rohdüse(n)" genannt) und auch der Innenseite (zweite Rohrdüse(n), im Folgenden auch "innere Rohrdüse(n)" genannt) des zu dezellularisierenden Organs/Gewebe ausüben. Durch diese intensive Verwirbelung wird der Dezellularisierungsvorgang erheblich beschleunigt. Die Verwirbelungen sorgen hierbei nicht allein für ein schnelles Lösen von Zellmaterial sondern auch für einen schnellen Abtransport von Zellmaterial, und für ein erleichtertes Eindringen der Reinigungslösung (z.B. SDS-Lösung) in die Matrix.

Des Weiteren sorgen die Verwirbelungen im Aktivkohle-Waschschritt (siehe unten) für einen optimalen Abtransport des an extrazellulärer Matrix angelagerten SDS.

Ferner wird der oben benutzte Begriff "stark" im Zusammenhang mit laminaren und/oder turbulenten Strömungen über die Flussraten beim Zuführen der Reinigungsflüssigkeit in die Rohrdüsen bestimmt. Die Flussgeschwindigkeit wird so eingestellt, dass die Reinigungsflüssigkeit mit einem großen Strömungsimpuls auf die (je nach Position der Rohrdüse: äußere oder innere) Oberfläche des zu dezellularisierenden Organs oder Gewebes trifft und/oder eine intensive Verwirbelung der verwendeten Flüssigkeiten am zu dezellularisierenden Organ/Gewebe erreicht wird. Dabei kann die Flussrate jedoch sehr unterschiedlich, abhängig vom verwendeten Rohrdüseninnendurchmesser, sowie dem zu dezellularisierenden Organ/Gewebe sein. Somit sind bei kleinen Organen/Geweben (beispielsweise bis 10 g, z.B. Organe von Insekten, kleinen Fischen, kleinen Vögeln, kleinen Reptilien, kleinen Säugetieren, beispielsweise Nagern wie etwa Mäusespeiseröhre, Mäuseherz, Mäuseniere ...) und Verwendung kleiner Rohrdüseninnendurchmesser (z.B. µm/mm-Bereich) sehr kleine Flussraten nötig (beispielsweise < 100 µl/min). Diese geringen Flussraten lassen sich beispielsweise durch den Einsatz anderer Flüssigkeitsförderpumpen oder durch das Einbringen von Regelventilen realisieren. Größere Organe (beispielsweise ab 10 g, z.B. Organe von großen Fischen, großen Vögeln, großen Reptilien, großen Säugetieren wie etwa Mensch, Schwein, Elefant; also z.B. Schweineherz, Schweineleber ...) bedingen unter Verwendung großer Rohrdüseninnendurchmesser (z.B. mm/cm-Bereich) eine entsprechend große Flussrate. Dabei liegen die Flussraten für weiche und/oder kleinere Organe/Gewebe zwischen 0,001 ml/min und 200 ml/min. Organe größerer Spezies wie dem Schwein oder Menschen erfordern hierbei Flussraten zwischen 200 ml/min und 10.000 ml/min.

In einer Ausführungsform des erfindungsgemäßen Verfahrens sind die zweiten Rohrdüsen jeweils so konfiguriert, dass ein Organ über die Länge der zweiten Rohrdüse aufziehbar ist, und jedes der zu dezellularisierenden Organe oder Gewebe ist entlang je einer der zweiten Rohrdüsen aufgezogen.

In einer Ausführungsform des Verfahrens weisen die zweite(n) Rohrdüse(n) jeweils einen Abfluss auf, der in den Innenraum des Reaktors mündet, wobei die Organe oder Gewebe jeweils so über die Rohrdüse aufgezogen sind, dass der Abfluss nicht durch das Organ oder Gewebe verdeckt ist.

In einer alternativen Ausführungsform des Verfahrens weisen die zweite(n) Rohrdüse(n) jeweils an der dem Zufluss gegenüberliegenden Ende einen weiteren Zufluss auf, durch den ebenfalls von außerhalb des Reaktors Flüssigkeit zuführbar ist, wobei diese weiteren Zuflüsse in Schritt (1a) berücksichtigt werden.

Es gibt jedoch auch Gewebestückchen oder Gewebe, bei denen ein Einfädeln von Rohrdüsen nicht möglich ist (z.B. Bänder, Sehnen, Gewebenetze oder "lappenartige" Strukturen). In diesem Fall werden vorzugsweise keine zweiten Rohrdüsen verwendet. An Stelle dessen werden die Gewebestückchen oder Gewebe an einer oder mehreren geeigneten Halterung(en), etwa Haken, die vorzugsweise an einer oberen Innenseite des Reaktors befestigt sind, in den Reaktor eingehangen oder befestigt. Im Betrieb werden die so im Reaktor positionierten Gewebestückchen oder Gewebe dann durch die ersten Rohrdüsen mit der Reinigungslösung (Dezellularisierungslösung) - unter Erzeugung von starken Strömungsimpulsen und/oder Verwirbelungen an der Oberfläche dieser Gewebestückchen oder Gewebe - umspült:

In einer weiteren Ausführungsform des erfindungsgemäßen Verfahrens ist jedes der zu dezellularisierenden Organe oder Gewebe zwischen den Rohrdüsen positioniert. Vorzugsweise erfolgt dabei die Positionierung der Organe mittels im Innenraum des Reaktors, beispielsweise an der oberen Innenseite, angebrachter Halterungen wie etwa Haken.

In einer bevorzugten Ausführungsform des Verfahrens sind die Rohrdüsen aus chemisch stabilen Werkstoffen gefertigt. Dabei ist vorzugsweise mindestens eine Rohrdüse aus einem festen Material, beispielsweise Stahl, rostfreiem Stahl, Glas, Acrylglas oder anderen festen chemisch stabilen Kunstoffen, gefertigt, und/oder mindestens eine Rohrdüse aus einem elastischen Material, beispielsweise elastischem Kunststoff, gefertigt und so konfiguriert, dass die mindestens eine aus elastischem Material gefertigte Rohrdüse flexibel durch ein Organ oder Gewebe führbar ist. Vorzugsweise entsprechen die aus elastischem Material gefertigten Rohrdüsen zumindest einem Teil der zweiten Rohrdüsen.

In einer Ausführungsform des Verfahrens werden der Schritt (1a) des Pressens und der Schritt (1b) des Ansaugens der Reinigungslösung mit einer oder mehrerer Pumpe(n), vorzugsweise Liquidpumpe(n), durchgeführt. Dabei ist der Ausgang der Pumpe(n) über ein Schlauchsystem mit den Zuflüssen verbunden, und der Eingang der Pumpe(n) ist mittels eines Schlauchs oder Schlauchsystems mit dem Abfluss verbunden. Die Flussrate an den Zuflüssen der Rohrdüsen ist vorzugsweise jeweils auf den Bereich zwischen 0,001 und 10.000 ml/min, mehr bevorzugt auf den Bereich zwischen 0,001 und 200 ml/min für kleinere Organe oder 200 bis 10000 ml/min für größere Organe, meist bevorzugt auf 0,001 bis 44 ml/min für kleinere Organe oder 200 bis 1300 ml/min für größere Organe eingestellt. Die Abgrenzung zwischen kleineren und größeren Organen/Geweben kann hier, wie bereits oben, wieder bei einem Gewicht von 10 g erfolgen (also kleinere Organe/Gewebe: bis 10 g, größere Organe/Gewebe: ab 10 g, ein Organ/Gewebe von 10 g kann sowohl als kleineres als auch als größeres Organ/Gewebe behandelt werden).

Typischerweise besitzt die Liquidpumpe eine Förderleistung von 200 bis 1.300 ml/min, welche für große Organe oder Gewebe geeignet ist. Bei Bedarf (z.B. Organe oder Gewebe von Großtieren wie Elefanten) können jedoch mehrere Pumpenausgänge in einem Zufluss vereinigt werden, wodurch sich Flussraten bis 10.000 ml/min erreichen lassen. Für kleinere Organe oder Gewebe werden dagegen vorzugsweise Schlauchpumpen mit einer Förderleistung von 0,001 ml/min bis 44 ml/min je Kanal verwendet. Da mehrere Kanäle zur Verfügung stehen und diese bei Bedarf in einem Zufluss vereinigt werden können, wird ein Bereich von Flussraten zwischen 0,001 ml/min und 200 ml/min für kleine Organe oder Gewebe abgedeckt.

In einer Ausführungsform des Verfahrens wird das Verfahren mehrmals, vorzugsweise jeweils nach vorbestimmten Zeitintervallen wie beispielsweise einem Tag, unterbrochen, um die alte Reinigungslösung gegen neue Reinigungslösung auszutauschen. Dabei finden vorzugsweise 2 bis 10, mehr bevorzugt 4 bis 8, meist bevorzugt 6 Unterbrechungen statt.

In einer Ausführungsform des Verfahrens weist die Reinigungslösung Sodiumdodecylsulphat (SDS) auf, vorzugsweise in 0,6% - 1,4%-iger Konzentration, mehr bevorzugt in 0,8% - 1,2%-iger Konzentration, meist bevorzugt in 1%-iger Konzentration in Aqua dest.

In einer Ausführungsform des Verfahrens weist das Verfahren die zusätzlichen vorbereitenden folgenden Schritte auf:
(9a) Entnahme der Organe oder Gewebe von toten Körpern;
(9b) Säuberung der Organe oder Gewebe, beispielsweise von Fett oder Tunica adventitia;
(9c) Einbau der Organe oder Gewebe in den Reaktor durch Aufziehen der Organe oder Gewebe auf je eine der zweiten Rohrdüsen oder Führen der zweiten Rohrdüsen durch die Organe oder Gewebe;
(9d) Fixierung der Organe oder Gewebe an den Rohrdüsen;
(9e) Herstellen der Reinigungslösung;
(9f) Befüllen des Reaktors mit der Reinigungslösung;
(9g) Anschließen des Reaktors mittels eines geschlossenen Schlauchsystems an die Pumpe;
(9h) Einstellen und Starten der Pumpe;
wobei die Schritte (9a) bis (9h) dem Schritt (1a) vorangehen.

Die bisherige in der Literatur beschriebene Vorgehensweise zur Dezellularisierung von Geweben/Organen beinhaltet meist eine anschließende Spülung mit einer Reinigungsflüssigkeit. Die Reinigungsflüssigkeiten beinhalten dabei meist anionische Tenside im Gemisch (z.B. SDS/SDC (Sodiumdeoxycholat)) oder einzeln (z.B. SDS). Dabei kommen Gesamtkonzentrationen bis zu 5% anionisches Tensid zum Einsatz. Des Weiteren können auch Säuren, Basen, hyper- und hypotone Lösungen oder Enzyme zum Einsatz kommen, wobei jede Verfahrensweise mit spezifischen Nachteilen verknüpft ist. Die Verwendung von anionischen Tensiden wie SDS führen hierbei zu den besten Ergebnissen, sowohl in Bezug auf die dreidimensionale Struktur als auch die physikalischen Eigenschaften der zu dezellularisierenden Organe, Organsysteme oder Gewebe.

Bei der Verwendung von SDS ergeben sich Probleme hinsichtlich der Konzentration von zytotoxischen SDS-Rückständen in den vollständig dezellularisierten Geweben/Organen.

Bisherige Lösungsversuche liegen in der Verwendung geringerer Detergenz-Konzentrationen bzw. Gemischen verschiedener Detergenzien, sowie einer langen "Waschphase". Teilweise wird in wissenschaftlichen Publikationen überhaupt nicht auf die Restkonzentration von Detergenzien eingegangen. Eine Verlängerung der Waschphase führt in erster Linie zu einem größeren Zeitaufwand im Herstellungsprozess. Zudem werden größere Mengen an Substanzen benötigt. Eine lange Waschphase kann die Gabe antibiotischer Substanzen mit sich bringen, um ein bakteriellen Befall des Gewebes/Organs zu verhindern (vgl. z.B. S. Caamaño, A. Shiori, S. H. Strauss, E. C. Orton: "Does sodium dodecyl sulfate wash out of detergent-treated bovine pericardium at cytotoxic concentrations"; J. Heart Valve: Dis., Januar 2009, 18(1):101-5; S. Cebotari, I. Tudorache, T. Jaekel, A. Hilfiker, S. Dorfman, W. Ternes, A. Haverich, A. Lichtenberg: "Detergent decellularization of heart valves for tissue engineering: toxicological effects of residual detergents on human endothelial cells", Artif Organs, März 2010, 34(3):206-10).

Bevorzugte Ausführungsformen der Erfindung ermöglichen daher ein effektives und schnelles Auswaschen von während des Herstellungsprozesses an die Matrix angelagerter, im Allgemeinen zytotoxischer Reinigungslösung (etwa SDS) aus der Matrix. Die erfindungsgemäße Anordnung der "Rohrdüsen" im Reaktor ermöglicht neben der Optimierung des Dezellularisierungsprozesses auch die Optimierung des Waschprozesses.

In einer bevorzugten Ausführungsform des Verfahrens weist das Verfahren die folgenden zusätzlichen weiteren Schritte auf:
(10a) Beseitigen der an den extrazellulären Matrizen gebundenen Reinigungslösung;
(10b) Entnehmen der dezellularisierten Organe oder Gewebe (extrazellulären Matrizen) aus dem Reaktor;
   und den folgenden jeweils optionalen Schritten:
(10c) Messen der Konzentration der verbliebenen Reinigungslösung in den dezellularisierten Organen oder Geweben (extrazellulären Matrizen); und/oder
(10d) Überführen der extrazellulären Matrizen in Reaktionsgefäße und anschließendem Befüllen der Reaktionsgefäße mit phosphatgepufferter Salzlösung (PBS); und/oder
(10e) Sterilisation der extrazellulären Matrizen mit Gammastrahlen, wobei die Energiedosis vorzugsweise 20 bis 30 kGy, mehr bevorzugt 23 bis 27 kGy, meist bevorzugt 25 kGy beträgt; und/oder
(10f) Lagerung der extrazellulären Matrizen bei 1° bis 14° C, vorzugsweise bei 2° bis 10° C, mehr bevorzugt bei 3° bis 7° C, meist bevorzugt bei 4° C.

In einer bevorzugten Ausführungsform des Verfahrens weist Schritt (10a) des Beseitigens der an den extrazellulären Matrizen gebundenen Reinigungslösung die folgenden Schritte auf:
(11a) Verbinden des Reaktors, einer ersten Pumpe, einem Reservoir, einem oder mehreren Reinigungsfiltern, die sich im Reservoir befinden und jeweils einen Zulauf sowie ein Überlaufventil oder ein Loch aufweisen, und einer zweiten Pumpe mittels eines Schlauchsystems so, dass:
   (i) der Abfluss des Reaktor durch einen ersten Schlauch mit dem Eingang der ersten Pumpe verbunden ist,
   (ii) der Ausgang der ersten Pumpe durch einen zweiten Schlauch oder ein weiteres Schlauchsystem mit dem Zufluss/den Zuflüssen des/der Reinigungsfilter(s) verbunden ist,
   (iii) das Innere des Reservoirs durch einen dritten Schlauch mit dem Eingang der zweiten Pumpe verbunden ist, und
   (iv) der Ausgang der zweiten Pumpe durch einen vierten Schlauch mit dem Zufluss oder den Zuflüssen des Reaktors verbunden ist;
(11b) Befüllen des Reservoirs mit einer Waschflüssigkeit, vorzugsweise Aqua dest;
(11c) Einstellen der Flussraten an der ersten Pumpe und an der zweiten Pumpe auf einen gleichen Wert;
(11d) Starten der Pumpen.

Dabei kann die erste Pumpe ein System von mehreren parallel zusammengeschlossenen Pumpen aufweisen; und die zweite Pumpe kann ein System von mehreren parallel zusammengeschlossenen Pumpen aufweisen. Vorzugsweise ist die erste Pumpe und/oder die zweite Pumpe eine Liquidpumpe. Ferner ist vorzugsweise die Flussrate an den jeweiligen Zuflüssen der Rohdüsen jeweils auf den Bereich zwischen 0,001 und 10.000 ml/min, mehr bevorzugt auf den Bereich zwischen 0,001 und 200 ml/min für kleinere Organe oder 200 bis 10.000 ml/min für größere Organe, meist bevorzugt auf 0,001 bis 44 ml/min für kleinere Organe oder 200 bis 1.300 ml/min für größere Organe eingestellt. Die Abgrenzung zwischen kleineren und größeren Organen/Geweben kann hier, wie bereits oben, wieder bei einem Gewicht von 10 g erfolgen (also kleinere Organe/Gewebe: bis 10 g, größere Organe/Gewebe: ab 10 g, ein Organ/Gewebe von 10 g kann sowohl als kleineres als auch als größeres Organ/Gewebe behandelt werden).

In einer Ausführungsform des Verfahrens weisen die Reinigungsfilter jeweils auf: ein Reaktionsgefäß, das mit einem Adsorptionsmittel befüllt ist; einen Deckel, mit dem das Reaktionsgefäß verschlossen ist, wobei der Deckel aufweist: einen Zulauf, beispielsweise ein Loch, und ein Überlaufventil, einen Abfluss oder ein Loch.

In einer bevorzugten Ausführungsform des Verfahrens weist das Adsorptionsmittel Aktivkohle und/oder zumindest einen der folgenden Stoffe auf: Zeolithe, Kunstharze, Tonmineralien, Aluminiumoxid, Holz, Sepharose, Silikatverbindungen. Vorzugsweise beträgt das Fassungsvermögen des Reaktionsgefäßes 20 bis 70 ml, und das Reaktionsgefäß ist mit 13 bis 47 g Aktivkohle befüllt. Mehr bevorzugt beträgt das Fassungsvermögen des Reaktionsgefäßes 30 bis 60 ml, und das Reaktionsgefäß ist mit 20 bis 40 g Aktivkohle befüllt. Meist bevorzugt beträgt das Fassungsvermögen des Reaktionsgefäßes 50 ml, und das Reaktionsgefäß ist mit 33 g Aktivkohle befüllt ist.

Die Porengröße der Aktivkohle kann zwischen 1 nm und 1 µm (Durchmesser) liegen, die Dichte zwischen 0,2 g/cm³ bis 0,6 g/cm³. Die Form kann je nach Anwendung von Pulver, Korn, Kugel, Stäbchen bis Platte variieren. Außer Aktivkohle können unterschiedlichste Zeolithe (z.B. A, X, Y, L, Mordenit, ZSM 5, ZSM 11), Kunstharze, Tonmineralien, Aluminiumoxid, Holz, Sepharose oder Silikatverbindungen zur Bindung der Reinigungsflüssigkeit (z.B. SDS) eingesetzt werden.

In einer Ausführungsform des Verfahrens wird die Waschflüssigkeit im Reservoir und/oder im Reaktor für einen Zeitraum von mehreren Tagen, vorzugsweise 10 bis 30 Tagen, mehr bevorzugt 15 bis 25 Tagen, meist bevorzugt 20 Tagen, zumindest einmal täglich durch neue Waschflüssigkeit ausgetauscht. Vorzugsweise wird beim Austauschen die in den Schläuchen, eventuell vorhandenen Schlauchdüsen etc. und sonstigen Bereichen des Systems verbliebene Waschflüssigkeit verworfen. Dabei erfolgt die Entnahme der dezellularisierten Organe oder Gewebe (extrazellulären Matrizen) aus dem Reaktor gemäß Schritt (10b) nach dem genannten Zeitraum; und vorzugsweise wird/werden während des genannten Zeitraums eine oder mehrere Messung(en) der Konzentration der in den extrazellulären Matrizen verbliebenen Reinigungslösung durchgeführt, wobei nach Messung einer Konzentration von weniger als einem vorbestimmten Schwellenwert, beispielsweise von weniger als 0,7 µmol SDS pro Gramm Organ oder Gewebe (Trockengewicht) (entspricht 0,202 mg SDS pro Gramm Organ oder Gewebe (Trockengewicht)) Schritt (10a) des Beseitigens der an den extrazellulären Matrizen gebundenen Reinigungslösung beendet werden kann.

In der Literatur sind sehr unterschiedliche Angaben zu toxischen SDS (Sodiumdodecylsulfat)-Konzentrationen zu finden. Die Angaben reichen von 177 µmol bis 10 µmol.

Mit der Waschmethode der oben beschriebenen Ausführungsform der Erfindung wird eine Rest-SDS-Konzentration von 0,12 mg SDS/g extrazellulärer Matrix (Trockengewicht) oder weniger erreicht. Dies entspricht einem Wert von 0,42 µmol SDS/g Organ oder Gewebe (Trockengewicht).

Als Schwellenwert für eine Obergrenze von verbliebenem SDS wird hier 0,7 µmol SDS/g Organ oder Gewebe (Trockengewicht) angesetzt. Beispielsweise könnten bei einem durchschnittlichen Wassergehalt von 92,17 ± 1,71% (Speiseröhre) bei einem Schwellenwert von 0,7 µmol/g (Trockengewicht) und 10 µmol als toxische Grenze ca. 175 g Organ oder Gewebe unbedenklich als Spenderorgan implantiert werden.

Mit den experimentell gemessenen Werten (0,42 µmol/g) könnten unbedenklich ca. 297 g Organ oder Gewebe, beispielsweise Speiseröhre, implantiert werden. Die lokale SDS-Konzentration am Implantationsort liegt dann, durch die Verdünnung mit Körperflüssigkeiten, ein Vielfaches unter der in der Literatur angegebenen toxischen SDS-Konzentration.

In einer Ausführungsform des Verfahrens weist das Messen der Konzentration der verbliebenen Reinigungsflüssigkeit in den extrazellulären Matrizen die folgenden Schritte aufweist:
(15a) Bereitstellen einer Reaktionsflüssigkeit, die Chloroform, vorzugsweise 4 ml Chloroform, und Methanol, vorzugweise 2 ml Methanol (2%-ig), umfasst;
(15b) Geben von Waschflüssigkeit, vorzugsweise 4 ml Waschflüssigkeit, oder homogenisierter Matrix, vorzugsweise 1 g homogenisierter Matrix (Trockengewicht; in flüssigem Stickstoff homogenisiert und in 4 ml Aqua dest gelöst), zur Reaktionsflüssigkeit;
(15c) Zugeben einer Methylenblaulösung, vorzugsweise von 500 µl einer 0,01 %-Methylenblaulösung;
(15d) intensives Mischen, vorzugsweise für 1 min;
(15e) Abwarten von mindestens 5 min, vorzugsweise 10 min;
(15f) photometrisches Bestimmen der Absorption der Chloroformphase mit Hilfe einer zuvor erstellten Eichkurve der Konzentration der Reinigungsflüssigkeit, vorzugsweise bei einer Wellenlänge von 620 bis 660 nm, mehr bevorzugt bei einer Wellenlänge von 630 bis 650 nm, meist bevorzugt bei einer Wellenlänge von 640 nm.

Ein weiterer Aspekt der Erfindung betrifft einen Reaktor gemäß Patentanspruch 16 zur Dezellularisierung von Organen oder Geweben, wobei der Reaktor aufweist: mindestens eine erste, äußere Rohrdüse und mindestens eine zweite, innere Rohrdüse, wobei die Rohrdüsen im Innenraum des Reaktors montiert sind, die zweite, innere Rohrdüse derart konfiguriert ist, dass das Organ oder Gewebe darauf aufziehbar ist und in die Rohrdüsen von außerhalb des Reaktors durch einen Zufluss Flüssigkeit zugeführt werden kann zur inneren und äußerlichen Behandlung der zu dezellularisierenden Organe oder Gewebe, und einen Abfluss.

In einer Ausführungsform des erfindungsgemäßen Reaktors sind die zweiten Rohrdüsen jeweils so konfiguriert sind, dass ein Organ über die Länge der zweiten Rohrdüse aufziehbar ist.

Ein weiterer Aspekt der Erfindung betrifft ein System zur Dezellularisierung von Organen oder Geweben, wobei das System aufweist: den erfindungsgemäßen Reaktor zur Dezellularisierung von Organen oder Geweben; eine Pumpe, vorzugsweise eine Liquidpumpe; wobei: der Ausgang der Pumpe über ein Schlauchsystem mit den Zuflüssen der des Reaktors verbunden ist; und der Eingang der Pumpe mittels eines Schlauchs mit dem Abfluss des Reaktors verbunden ist.

Ein weiterer Aspekt der Erfindung betrifft ein System zum Beseitigen von an dezellularisierten Organen oder Geweben (extrazellulären Matrizen) gebundener Reinigungslösung, wobei das System aufweist: den erfindungsgemäßen Reaktor zur Dezellularisierung von Organen oder Geweben; eine erste Pumpe, vorzugsweise eine Liquidpumpe; ein Reservoir; ein oder mehrere Reinigungsfilter, die sich im Reservoir befinden und jeweils einen Zulauf sowie ein Überlaufventil oder ein Loch aufweisen; eine zweite Pumpe, vorzugsweise eine Liquidpumpe; wobei der Reaktor, die erste Pumpe, das Reservoir, der oder die mehreren Reinigungsfilter und die zweite Pumpe mittels eines Schlauchsystems so verbunden sind, dass:
(i) der Abfluss des Reaktor durch einen ersten Schlauch mit dem Eingang der ersten Pumpe verbunden ist,
(ii) der Ausgang der ersten Pumpe durch einen zweiten Schlauch oder ein weiteres Schlauchsystem mit dem Zufluss/den Zuflüssen des/der Reinigungsfilter(s) verbunden ist,
(iii) das Innere des Reservoirs durch einen dritten Schlauch mit dem Eingang der zweiten Pumpe verbunden ist, und
(iv) der Ausgang der zweiten Pumpe durch einen vierten Schlauch mit dem Zufluss oder den Zuflüssen des Reaktors verbunden ist.

Dabei kann die erste Pumpe ein System von mehreren parallel zusammengeschlossenen Pumpen aufweisen; und die zweite Pumpe kann ein System von mehreren parallel zusammengeschlossenen Pumpen aufweisen.

In einer Ausführungsform des erfindungsgemäßen Systems zum Beseitigen von an dezellularisierten Organen oder Geweben (extrazellulären Matrizen) gebundener Reinigungslösung weisen die Reinigungsfilter jeweils auf: ein Reaktionsgefäß, das mit einem Adsorptionsmittel befüllt ist; einen Deckel, mit dem das Reaktionsgefäß verschlossen ist, wobei der Deckel aufweist: einen Zulauf, beispielsweise ein Loch, und ein Überlaufventil, einen Abfluss oder ein Loch.

Dabei kann das Adsorptionsmittel Aktivkohle aufweisen. Dabei beträgt vorzugsweise das Fassungsvermögen des Reaktionsgefäßes 20 bis 70 ml. Mehr bevorzugt beträgt das Fassungsvermögen des Reaktionsgefäßes 30 bis 60 ml. Meist bevorzugt beträgt das Fassungsvermögen des Reaktionsgefäßes 50 ml.

Das Adsorptionsmittel kann zusätzlich oder alternativ auch Zeolithe, Kunstharze, Tonmineralien, Aluminiumoxid, Holz, Sepharose und/oder Silikatverbindungen aufweisen.

Es zeigen:
- Fig. 1:: SDS in der Waschflüssingkeit. SDS-Konzentration in der Waschlösung von Aqua dest und Aktivkohle behandelten Speiseröhren. ***p<0,001 vs. Aqua dest.
- Fig. 2:: Verbliebenes SDS im Trockengewebe. Menge des an dezellularisierte Matrix gebundenen SDS nach den unterschiedlichen Waschmethoden im Vergleich zu der SDS-Konzentration vor der Waschphase. A: Absolute Menge an SDS pro Gramm Gewebe (Trockengewicht). B: Vergleich der prozentualen SDS-Konzentration nach Aktivkohlebehandlung gegenüber alleinigem Waschen mit Aqua dest. ⁺⁺⁺p<0,001 vs. vor Waschphase, ***p<0,001 vs. Aqua dest.
- Fig. 3:: Schematische Anordnung der vier äußeren Rohrdüsen (36a - 36d) im Reaktionsgefäß
- Fig. 4:: Schematischer Aufbau der inneren Rohrdüse mit fixierter Speiseröhre
- Fig. 5:: Schematische Darstellung des Reaktionsgefäßes in der Seitenansicht
- Fig. 6:: Aufbau des Aktivkohlefilters
- Fig. 7:: Aufbau des Systems zum Beseitigen von an dezellularisierten Organen oder Geweben (extrazellulären Matrizen) gebundener Reinigungslösung mit Reaktor, Pumpen und Aktivkohlefilter und Reservoir
- Fig. 8:: Schematische Darstellung einer Schlauchdüse. Auf der linken Seite ist eine komplette Schlauchdüse zu sehen. An beiden Enden wird unter hohem Druck Flüssigkeit in den Schlauch befördert. In der Vergrößerung auf der rechten Seite sind die Lochbohrungen (Düsen, weiße Punkte) der Schlauchdüse zu erkennen.

Die Erfindung wird nachstehend anhand von Beispielen und der Zeichnung erläutert.

Im folgenden sei am Beispiel einer Speiseröhre detailliert beschrieben, wie ein Organ gemäß einer Ausführungsform des erfindungsgemäßen Verfahrens dezellularisiert (1. Phase) und das dezellularisierte Organ anschließend wieder von der Reinigungsflüssigkeit befreit werden kann (2. Phase). Im 2. Schritt wird dabei gemessen, wie viel Reinigungsflüssigkeit noch im dezellularisierten Organ verblieben ist. Diese Messung ist im Folgenden als 3. Phase beschrieben.

Es versteht sich, dass das Verfahren, wie es hier für eine Speiseröhre beschrieben ist, auch für andere Organe, Gewebe oder Organsysteme verwendet werden kann, wobei jedoch gegebenenfalls die Parameter bezüglich der Abmessungen, der Befüllung mit Aktivkohle usw. angepasst werden müssen oder andere Spezies zur Entnahme verwendet werden.

Ferner sind diejenigen Schritte, die nicht Schritte aus den Ansprüchen entsprechen, als optional zu betrachten.

### 1. Phase: Durchführung der Dezellularisierung von Speiseröhren

- Arbeitsplatz vorbereiten: Sterilbank einschalten, Arbeitsfläche desinfizieren, benötigte Arbeitsmaterialien ebenfalls desinfizieren/autoklavieren und unter Sterilbank stellen, Hände desinfizieren
- Entnahme der Speiseröhre(n) von toten Körpern, z.B. Schweinen, vorzugsweise im OP, und Transport in antibiotischer Lösung
- Säuberung der Speiseröhre(n) von allen äußeren Gewebsresten (z.B. Fett, Tunica adventitia), vorzugsweise mittels kleiner scharfer OP-Schere und Pinzette zum Festhalten
- Entfernen eines etwa 1 cm breiten Ringes der Muskularis an den beiden Enden der Speiseröhre(n)
- Einbau der Speiseröhre(n) in den Reaktor durch Aufziehen der Speiseröhre(n) auf die inneren Rohrdüse(n) und anschließend auf das/die jeweiligen, unterhalb der Rohrdüse(n) montierte(n) Ablaufrohr(e) im Reaktor
- Fixierung der Speiseröhre(n) an den Anschlussstücken (z.B. mittels chirurgischem Nahtmaterial, Gewebekleber; Fixierung sollte sehr fest sein und nach Möglichkeit kein Muskel mit eingebunden werden)
- Herstellen einer 1%-igen SDS-Lösung (in Aqua dest)
- Befüllen des Reaktors mit 1%-iger SDS-Lösung
- Anschluss des Reaktors an ein geschlossenes Schlauchsystem einer Liquidpumpe
- Start und Einstellen der Liquidpumpe auf eine Flussrate von ca. 400 ml/min
- SDS-Lösung wird aus dem Reaktor angesaugt und über die Pumpe durch die Rohrdüse(n) im Organlumen, sowie den Rohrdüsen im Reaktorraum gepresst (Fig. 3 - 5)
- die Speiseröhre(n) wird/werden somit innen (Lumen) und außen (Muskularis) gleichmäßig mit der 1%-igen SDS-Lösung benetzt, wobei die Speiseröhre(n) starken laminaren und/oder turbulenten Strömungen der SDS-Lösung ausgesetzt ist/sind
- 1-mal täglich erfolgt die Unterbrechung des Dezellularisierungsvorgangs um die SDS-Lösung aus dem Reaktor, Schlauchdüsen und den Schläuchen zu entfernen und gegen neu hergestellte 1%-ige SDS-Lösung auszutauschen
- Ergebnis: nach etwa 7 Tagen ist/sind die Speiseröhre(n) zellfrei.

### 2. Phase: Beseitigung von "an der extrazellulären Matrix gebundenem SDS"

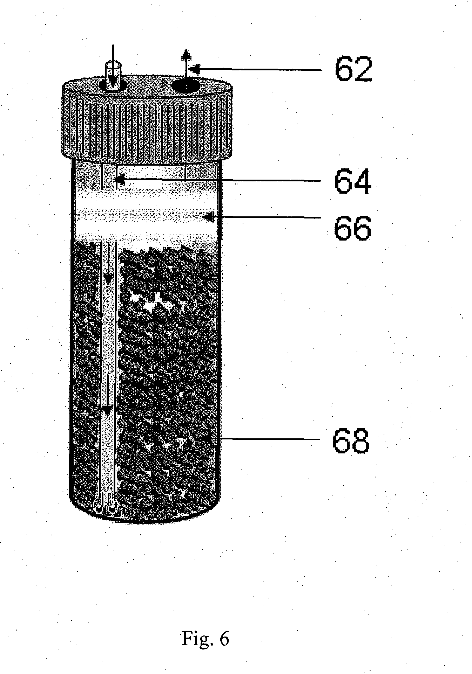

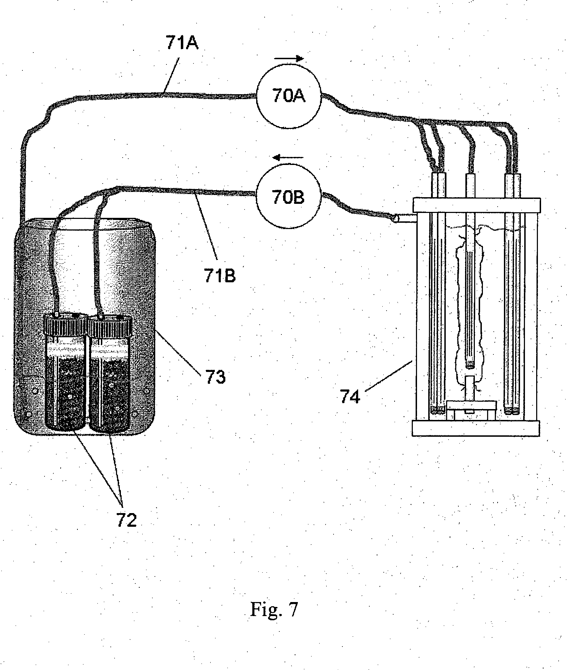

- zwei Reaktionsgefäße mit je 50 ml Fassungsvermögen werden mit jeweils 33 g Aktivkohle befüllt, mit einem Deckel verschraubt (im Deckel befindet sich ein Loch für den Zulauf der Pumpe und ein Loch als Überlaufventil, vgl. Fig. 6), in ein ausreichend großes Reservegefäß überführt und über ein Schlauchsystem mit den Abläufen einer ersten Liquidpumpe (beispielsweise der Liquidpumpe, die während der Ausführung des 1. Schritts mit den Rohrdüsen verbunden war), verbunden
- Befüllung des Reservegefäßes mit Aqua dest (entsprechend einer Aktivkohlekonzentration von 0,025 g/ml bis 0,15 mg/ml Aqua dest)
- eine zweite Liquidpumpe wird mit dem Zufluss über ein Schlauchsystem mit dem Reservegefäß und der Abfluss mit den Rohrdüsen verbunden (zum Gesamtaufbau vgl. Fig. 7)
- die zweite Liquidpumpe wird ebenfalls auf eine Flussrate von ~400 ml/min eingestellt
- nach Start beider Pumpen wird das Aqua dest durch die in den 50 ml Reaktionsgefäßen befindliche Aktivkohle gepresst, das somit gereinigte Aqua dest läuft dabei durch das Überlaufventil in das Reservegefäß
- Aqua dest (im Reservegefäß und Reaktor) sowie Aktivkohle in den Reaktionsgefäßen werden 1-mal täglich für den Zeitraum von 20 Tagen gewechselt und das jeweils verbleibende Aqua dest im Reaktor, Schlauchdüsen und Schläuchen verworfen
- nach 20 Waschtagen erfolgt die Entnahme der Organe (z.B. Speiseröhren); Lösung ablassen, Reaktor öffnen, Fixierung lösen
- Speiseröhren (bestehen nun ausschließlich aus extrazellulärer Matrix) in 50 ml Reaktionsgefäße (Plastikröhrchen) überführen und mit phosphatgepufferter Salzlösung (PBS) befüllen
- Sterilisation mit Gammastrahlen bei 25 kGy
- Lagerung bei 4 °C.

### 3. Phase: Messen der SDS-Konzentration

- 4 ml Waschlösung bzw. 1 g homogenisierte extrazelluläre Matrix (Trockengewicht; in flüssigem Stickstoff homogenisiert und in 4 ml Aqua dest gelöst) werden zur Reaktionsflüssigkeit gegeben
- Reaktionsflüssigkeit besteht aus 4 ml Chloroform und 2 ml Methanol (2 %-ig)
- Zugabe von 500 µl einer 0,01 % Methylenblaulösung
- intensives Mischen für den Zeitraum von 1 min
- nach 10 min wird die Absorption der Chloroformphase bei einer Wellenlänge von 640nm photometrisch bestimmt und anhand einer zuvor erstellten Eichkurve die SDS-Konzentration der Probe bestimmt.

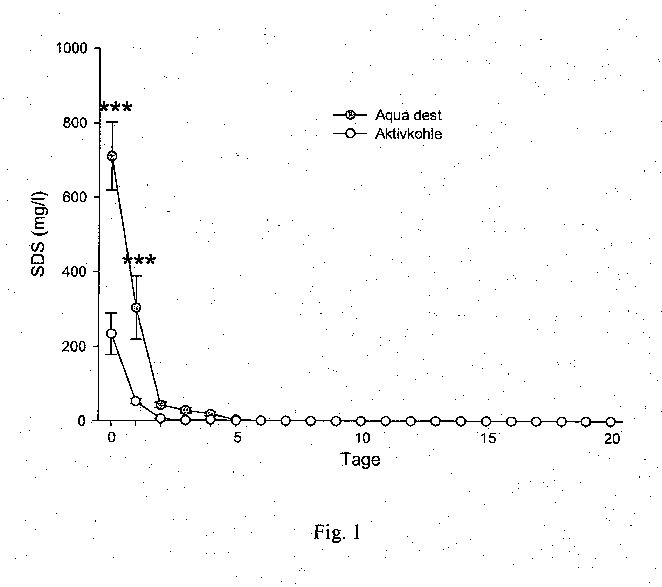

Nach bereits 5 Tagen ist die SDS-Konzentration im Spülmedium unter Verwendung von Aqua dest und Aktivkohle nahezu entfernt (vgl. Fig. 1). Dabei ist auffällig, dass ein Waschen mit Aktivkohle zu einer signifikant schnelleren Entfernung des SDS aus der Waschlösung und somit auch aus der extrazellulären Matrix (durch Diffusion) führt (vgl. Fig. 1).

Wird der SDS-Gehalt der extrazellulären Matrix (an Matrix gebundenes SDS) nach der Waschphase gemessen, beobachtet man eine signifikante Reduktion des SDS-Gehaltes der mit Aktivkohle gewaschenen Matrizen gegenüber den mit Aqua dest gewaschenen. Durch das Verwenden der Aktivkohle können zytotoxische SDS-Rückstände effektiver beseitigt werden.

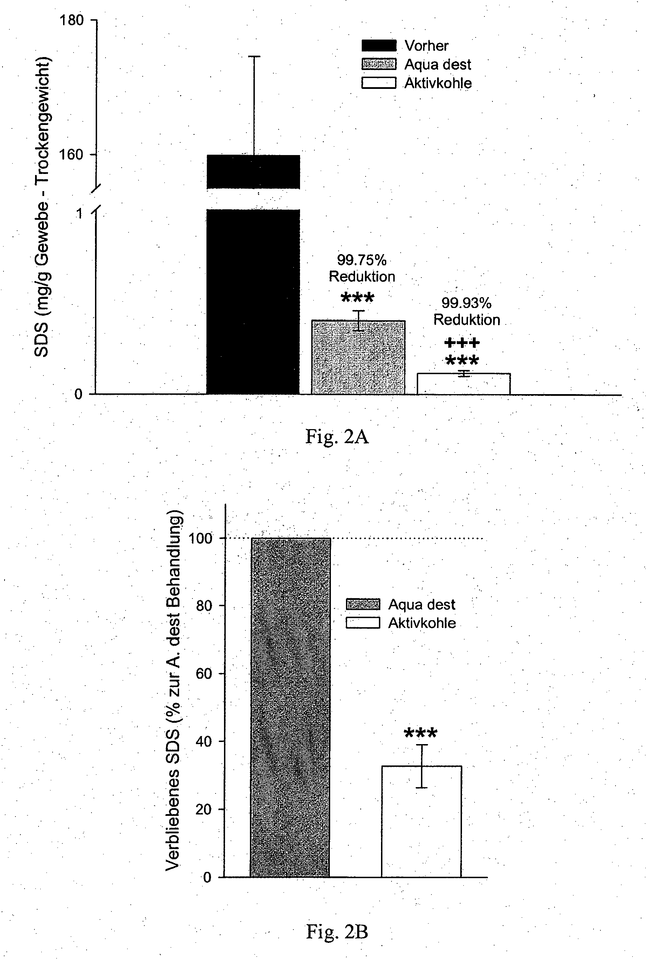

Nach der Gesamtdauer von 20 Tagen ist 99,7 % (Aqua dest) bis 99,9 % (Aktivkohle) des ursprünglich an der extrazellulären Matrix gebundenen SDS entfernt (vgl. Fig. 2A). Dabei wird der Wascheffekt unter Verwendung von Aktivkohle signifikant begünstigt. Unter Verwendung der Aktivkohle findet man nur ⅓ der SDS-Konzentration im Vergleich zu den mit Aqua dest gewaschenen Speiseröhren (vgl. Fig. 2B).

Nachfolgend seien schematisch Ausführungsformen des erfindungsgemäßen Reaktors, der Rohrdüse bzw. Schlauchdüse, des Filters sowie des erfindungsgemäßen Systems zum Beseitigen von an dezellularisierten Organen oder Geweben (extrazellulären Matrizen) beschrieben.

Figur 3 zeigt im Querschnitt schematisch den Aufbau des erfindungsgemäßen Reaktors in einer möglichen Ausführungsform. Innerhalb der Reaktionsgefäßwand 30 sind vier äußere Rohrdüsen 36a, 36b, 36c, 36d angeordnet. In dem Reaktor ist zudem eine innere Rohrdüse 34 so angeordnet, dass sie von dem äußeren Rohrdüsen 36a - 36d umgeben ist. Über die innere Rohrdüse 34 ist das Organ oder Gewebe 32 aufgezogen, das dezellularisiert werden soll. Dieses Gewebe oder Organ 32 ist hier schematisch als Kreis dargestellt. Durch geeignete Zuflüsse (nicht dargestellt) kann sowohl der inneren Rohrdüse 34 als auch den äußeren Rohrdüsen 36a - 36d Reinigungsflüssigkeit zugeführt werden. In den Wänden der Rohrdüsen 34, 36a - 36d befinden sich jeweils eine Vielzahl an Austrittsöffnungen oder Düsen, durch die Reinigungsflüssigkeit aus dem Inneren der Rohrdüsen in den Innenraum des Reaktors strömen kann. Die Stärke der Strömungen, die aus den Löchern oder Düsen in der Wand einer Rohrdüse austreten, ist dabei bestimmt durch den Druck der Reinigungsflüssigkeit in der jeweiligen Rohrdüse. Der Druck wiederum ergibt sich dabei aus der Geometrie der betrachteten Rohrdüse und der Flussrate (Volumen/Zeit, beispielsweise in Einheiten von ml/min) bei der Zufuhr von Reinigungslösung. Die Zufuhr von Reinigungslösung in die Rohrdüsen geschieht üblicherweise mithilfe einer geeigneten Pumpe oder eines geeigneten Systems aus mehreren Pumpen; die Flussrate bei der Zufuhr von Reinigungslösung in die Rohrdüse kann daher durch ein Einstellen der Flussrate an der entsprechenden Pumpe oder den entsprechenden Pumpen eingestellt werden. Strömt die Reinigungsflüssigkeit aus den in der Wand einer Rohrdüse befindlichen Löchern oder Düsen, so ist die Richtung der durch ein bestimmtes Loch oder einer bestimmten Düse austretenden Strömung unmittelbar nach dem Austritt aus dem betrachteten Loch oder der betrachteten Düse typischerweise normal (orthogonal) zu der Fläche der Wand der Rohrdüse an der Stelle, an der sich das betrachtete Loch oder die betrachtete Düse befindet und aus der Rohrdüse heraus gerichtet. In Figur 3 ist dies schematisch durch die Pfeile 37 dargestellt, die sich an den Außenseiten der Wände der äußeren Rohrdüsen 36a - 36d sowie der inneren Rohrdüse 34 befinden. Da die Querschnitte der in Figur 3 gezeigten Rohrdüsen alle kreisförmig sind, ist die Strömung unmittelbar nach Austritt aus einem bestimmten Loch oder einer bestimmten Düse radial vom Mittelpunkt eines Querschnitts einer Rohrdüse hin nach außen gerichtet. Im Betrieb ist der Innenraum des Reaktors bereits mit Reinigungsflüssigkeit befüllt. Daher strömt die Reinigungsflüssigkeit beim Austritt aus den Rohrdüsen direkt in die bereits im Reaktor befindliche Reinigungsflüssigkeit und vermischt sich mit dieser. Je nach Druck oder Geschwindigkeit der aus den Rohrdüsen ausströmenden Reinigungsflüssigkeit kann diese sich entweder als laminare Strömung innerhalb der bereits im Reaktorinnenraum befindlichen Reinigungsflüssigkeit bewegen, oder es kommt zu turbulenten Verwirbelungen. In Figur 3 sind diese Verwirbelungen schematisch durch die gekrümmten Pfeile 38 dargestellt. Das entlang der inneren Rohrdüse 34 aufgezogene Organ oder Gewebe 32 ist somit einerseits den laminaren und/oder turbulenten Strömungen ausgesetzt, die durch das Ausströmen von Reinigungsflüssigkeit aus den äußeren Rohrdüsen 36a - 36d entstehen, und andererseits zugleich den laminaren und/oder turbulenten Strömungen ausgesetzt, die durch die aus der inneren Rohrdüse 34 austretenden Strömungen erzeugt werden. Durch den erfindungsgemäßen Aufbau des Reaktors lassen sich daher strömungsmechanische Prozesse erzielen, durch die das Gewebe oder Organ 32 besonders stark dem Einfluss der Reinigungsflüssigkeit exponiert ist.

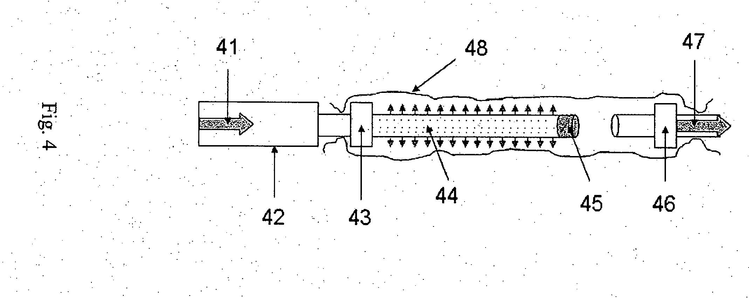

Figur 4 zeigt schematisch einen möglichen Aufbau der inneren Rohrdüse mit fixiertem Organ oder Gewebe 48, wie er im erfindungsgemäßen Reaktor benutzt werden kann. Die Rohrdüse weist ein Rohr 44, beispielsweise ein Plastikrohr, auf, in dessen Wand sich eine Vielzahl von Öffnungen, beispielsweise Bohrungen, oder Düsen befindet. Ein Ende des Rohres (in der Abbildung das linke Ende des Rohres 44) dient als Zufluss. Der Zufluss kann beispielsweise mit einem Schlauch 42 verbunden sein, der über das entsprechende Ende des Rohres 44 gesteckt ist. Die Richtung der einströmenden Reinigungsflüssigkeit ist hier durch den Pfeil 41 angedeutet. Am dem Zufluss gegenüberliegenden Ende 45 des Rohres 44 ist das Rohr verschlossen. Die in Figur 4 gezeigte Anordnung weist ferner einen Abfluss auf, durch den Reinigungsflüssigkeit in der durch den Pfeil 47 angedeuteten Richtung abfließen kann. Dieser Abfluss kann beispielsweise durch ein Rohr realisiert sein, das so angeordnet ist, dass seine gedankliche (d.h. virtuelle) Mittelachse identisch mit der gedanklichen Mittelachse der Rohrdüse 44 ist, wobei sich der Abfluss in einem gewissen Abstand zur Rohrdüse 44 befindet, jedoch an der Seite des Endes 45 der Rohrdüse 44 angeordnet ist. Der Abfluss weist ferner einen Gummiring 46 zur Fixierung des Gewebes oder Organs 48 auf. Ein weiterer Gummiring zur Fixierung des Organs oder Gewebes ist auch an der Rohrdüse 44 angeordnet, wobei der weitere Gummiring dabei so positioniert ist, dass er sich - in Flussrichtung gesehen - etwas unterhalb des Zuflusses 42, jedoch oberhalb der in der Wand der Rohrdüse 44 befindlichen Austrittsöffnungen oder Düsen befindet. Auf diese Weise kann das Organ oder Gewebe 48 einerseits durch den Gummiring 46 am Abfluss fixiert werden und andererseits durch den weiteren Gummiring 43 im oberen Bereich der Rohrdüse 44 fixiert werden. Wird nun Reinigungsflüssigkeit durch den Zufluss 42 in die Rohrdüse 44 gepresst, strömt die Reinigungsflüssigkeit durch die Austrittsöffnungen oder Düsen in der Wand der Rohrdüse 44 aus und gelangt in den Innenbereich des Organs oder Gewebes 48. Dabei kann es wieder zu einer Vielzahl von strömungsmechanischen Effekten, wie etwa Verwirbelungen oder Turbulenzen kommen, deren Stärke durch die Austrittsgeschwindigkeit aus den Austrittsöffnungen oder Düsen, den Druck im Inneren der Rohrdüse und/oder der Flussrate der durch den Zufluss 42 zugeführten Reinigungsflüssigkeit bestimmt wird. Auf diese Weise ist das Organ oder Gewebe 48 auch von innen der Reinigungsflüssigkeit ausgesetzt. Hat sich der Innenbereich des Organs oder Gewebes 48 mit Reinigungsflüssigkeit gefüllt, kann überschüssige Reinigungsflüssigkeit durch den Abfluss in Richtung des Pfeils 47 aus dem Innenbereich des Organs oder Gewebes 48 entweichen.

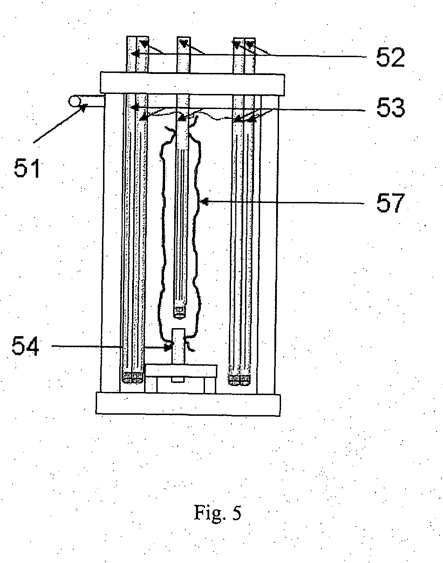

Figur 5 zeigt schematisch den Innenaufbau des erfindungsgemäßen Reaktors in einer möglichen Ausführungsform in einer Seitenansicht. Im Innenbereich des Reaktors befinden sich wieder fünf Rohrdüsen 53, die beispielsweise den äußeren Rohrdüsen 36a - 36d sowie der inneren Rohrdüse 34 aus Figur 3 entsprechen. Entlang einer inneren Rohrdüse ist ein Organ oder Gewebe 57, beispielsweise eine Speiseröhre, fixiert. Jede der fünf Rohrdüsen 53 besitzt einen Zufluss 52. Weiterhin verfügt der Reaktor über zwei Abflüsse: Ein Abfluss 51 ist dabei im oberen Bereich des Reaktors so angeordnet, dass Reinigungsflüssigkeit aus dem oberen Bereich des Innenraums des Reaktors, aber aus einem Bereich außerhalb des Organs oder Gewebes 57 abfließen kann. Ein weiterer Abfluss 54 befindet sich im Innenraum des Reaktors unterhalb der inneren Rohrdüse, um die das Gewebe oder Organ 57 aufgezogen ist, wobei diese Rohrdüse entsprechend verkürzt ist. Insbesondere ist das Organ oder Gewebe 57 so im Reaktor fixiert, dass sich eine Fixierung im oberen Bereich der inneren Rohrdüse befindet und sich eine weitere Fixierung des Organs oder Gewebes 57 an diesem weiteren Abfluss 54 befindet. Im Innenraum des Reaktors kann somit Reinigungsflüssigkeit aus dem Inneren des Gewebes oder Organs 57 in den Bereich außerhalb des Gewebes oder Organs 57 strömen. Die Zufuhr von Reinigungsflüssigkeit, die entstehenden Strömungen sowie die strömungsmechanischen Effekte auf das Organ oder Gewebe 57 sind bereits oben im Zusammenhang mit Figur 3 erläutert worden.

Figur 6 zeigt den Aufbau eines Aktivkohlefilters. Der Aktivkohlefilter weist ein Gefäß auf, das mit einem Verschluss, beispielsweise einem Deckel, verschlossen ist, in dem sich zwei Öffnungen befinden. Durch eine der Öffnungen (in der Figur links gezeichnet) wird ein als Zufluss dienendes Rohr 64 eingeführt, das sich fast über die gesamte Länge des Aktivkohlefilters in dessen Innenraum erstreckt, und nur am Ende etwas Freiraum lässt, so dass eine an diesem Ende ausströmende Flüssigkeit in das Innere des Aktivkohlefilters entweichen kann. Um den Zufluss 64 herum ist der Innenbereich des Aktivkohlefilters mit Aktivkohle 68 befüllt, wobei an der dem Deckel zugewandten Aktivkohlegrenzschicht eine engfasrige Filterschicht 66 (z.B. mehrere Schichten Watte und/oder Vlies) angebracht ist. Die zweite Öffnung 62 im Verschluss des Aktivkohlefilters dient als Abfluss oder Überlaufventil. Wird nun eine Flüssigkeit durch den Zufluss 64 dem Aktivkohlefilter zugeführt, kann die Flüssigkeit aus dem Aktivkohlefilter erst wieder austreten, nachdem sie den mit Aktivkohle 68 befüllten Bereich des Aktivkohlefilters passiert hat. In der Waschlösung enthaltene Reinigungslösung (z.B. SDS) bindet an der Aktivkohle und aus dem Abfluss oder Überlaufventil 62 tritt eine gesäuberte Flüssigkeit wieder aus.

Figur 7 zeigt den Aufbau des erfindungsgemäßen Systems zum Beseitigen von an dezellularisierten Organen oder Geweben (extrazellulären Matrizen) gebundener Reinigungslösung mit Reaktor 74, Pumpen 70A, 70B und Filter(n) 72 sowie Reservoir 73. Das System weist ein Reservoir 73 auf, in dem sich ein oder mehrere Filter 72 zum Reinigen der Waschlösung von darin enthaltenen Detergenzien (z.B. SDS) befinden. Bei diesen Filtern 72 kann es sich beispielsweise um die oben im Zusammenhang mit Figur 6 bereits beschriebenen Aktivkohlefilter handeln. Ferner umfasst das System den erfindungsgemäßen Reaktor 74 zur Dezellularisierung von Organen oder Geweben, beispielsweise in Ausführungsformen wie sie oben bereits im Zusammenhang mit den Figuren 3 bis 5 erläutert worden sind. Im Betrieb sind sowohl das Reservoir 73 als auch der Reaktor 74 bis zu einer gewissen Höhe der jeweiligen Innenräume mit Waschflüssigkeit, beispielsweise Aqua dest, befüllt. Ein Schlauch 71A, dessen erstes Ende so im Reservoir 73 gehalten wird, dass es sich in der darin befindlichen Waschflüssigkeit befindet, wird mit seinem zweiten Ende an den Eingang einer Pumpe 70A, beispielsweise einer Liquidpumpe, angeschlossen. Der Ausgang dieser Pumpe 70A wird mit einem Schlauch oder einem Schlauchsystem mit dem oder den Zuflüssen des erfindungsgemäßen Reaktors 74 verbunden. Der Abfluss des Reaktors wird mit einem Schlauch an den Eingang einer weiteren Pumpe 70B, die beispielsweise ebenfalls eine Liquidpumpe sein kann, verbunden. Der Ausgang dieser weiteren Pumpe 70B wird mit einem Schlauch oder Schlauchsystem 71B mit den Zuflüssen der Filter 72 verbunden. Im Betrieb werden nun die erste Pumpe 70A und die weitere Pumpe 70B so betrieben, dass Waschflüssigkeit aus dem Reservoir 73 mittels der ersten Pumpe 70A dem Reaktor 74 zugeführt wird, und dass Waschflüssigkeit aus dem Reaktor 74 mittels der weiteren Pumpe 70B dem oder den Filter(n) 72 zugeführt wird. Vorzugsweise werden die Flussraten der beiden Pumpen dabei identisch gewählt. Aus den Reinigungsfiltern 72 austretende gereinigte Waschflüssigkeit wird dann zunächst im Reservoir 73 aufgefangen. Die im Reservoir 73 befindliche Waschflüssigkeit wird dann über den Schlauch 71A dem Reaktor zugeführt, in dem an der extrazellulären Matrix gebundenes SDS ein oder mehrere Organe oder Gewebe mittels dieser Waschflüssigkeit entfernt wird, wobei die Waschflüssigkeit selbst mit SDS angereichert oder verunreinigt wird. Diese so mit SDS angereicherte oder verunreinigte Flüssigkeit wird dann mittels der weiteren Pumpe 70B wiederum den Filtern 72 zugeführt, womit der Zyklus von Neuem beginnt.

In Figur 8 wird schematisch eine Schlauchdüse 80 dargestellt. Schlauchdüsen sind im Gegensatz zu Rohrdüsen nicht aus festem Material gefertigt, sondern sind flexibel und können beispielsweise aus dünnen Kunststoffschläuchen gefertigt werden. Diese Flexibilität oder Biegsamkeit ermöglicht den Einsatz der Schlauchdüsen auch bei komplexen Organen wie etwa einem Herzen, wobei die Schlauchdüsen durch das gesamte Organvolumen, beispielsweise vom linken Vorhof zur linken Herzkammer über den rechten Vorhof zur rechten Herzkammer, geführt werden können, ohne ein eigentliches Kanülieren des Organs vorzunehmen. Ähnlich wie im Zusammenhang mit Figur 4 bereits für die Rohrdüse beschrieben, ist auch die Schlauchdüse 80 mit einer Reihe von Austrittsöffnungen oder Düsen versehen, die das Austreten von in die Schlauchdüse 80 gepresster Reinigungsflüssigkeit ermöglichen. Der durch das (virtuelle) Rechteck 82 markierte Teil 84 der Schlauchdüse 80 ist in dem durch das (virtuelle) Rechteck 82a markierten Bereich vergrößert dargestellt; schematisch ist dabei auch eine mögliche Anordnung der Austrittsöffnungen oder Düsen (hier als weiße Punkte dargestellt) eingezeichnet. Im einfachsten Falle stellen die Austrittsöffnungen auch hier wieder Lochbohrungen dar.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren auch wenn sie dort im Zusammenhang mit anderen Merkmalen gezeigt sind und/oder vorstehend nicht genannt sind.

Im Weiteren schließt der Ausdruck "umfassen" und Ableitungen davon andere Elemente oder Schritte nicht aus. Ebenfalls schließt der unbestimmte Artikel "ein" bzw. "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

## Patentansprüche

1. Verfahren zur Dezellularisierung von Organen oder Geweben in einem Reaktor, wobei der Reaktor aufweist:
mindestens eine erste, äußere Rohrdüse und mindestens eine zweite, innere Rohrdüse, wobei:
die Rohrdüsen im Innenraum des Reaktors montiert sind; und
in die Rohrdüsen von außerhalb des Reaktors durch einen Zufluss
Flüssigkeit zugeführt werden kann;
einen Abfluss;
wobei jedes der zu dezellularisierenden Organe oder Gewebe im Innenraum des Reaktors positioniert ist und auf eine zweite, innere Rohrdüse aufgezogen ist;
wobei sich im Reaktor eine Reinigungslösung befindet; und
wobei das Verfahren folgende Schritte aufweist:
(1a) Pressen einer Reinigungslösung mit Druck durch die jeweiligen Zuflüsse in die
ersten und zweiten Rohrdüsen zur inneren und äußerlichen Behandlung der zu dezellularisierenden Organe oder Gewebe;
(1b) Ansaugen von Reinigungsflüssigkeit durch den Abfluss aus dem Reaktor;
wobei:
der Druck in Schritt (1a) so gewählt ist, dass im Reaktor starke laminare und/oder turbulente Strömungen und Verwirbelungen in der Reinigungslösung auftreten; und
die Schritte (1a) und (1b) vorzugsweise gleichzeitig und für die Dauer einer vorbestimmten Zeit ausgeführt werden.

2. Verfahren nach Anspruch 1,
wobei die zweiten Rohrdüsen jeweils so konfiguriert sind, dass ein Organ über die Länge der zweiten Rohrdüse aufziehbar ist; und
wobei jedes der zu dezellularisierenden Organe oder Gewebe entlang je einer der zweiten Rohrdüsen aufgezogen ist.

3. Verfahren nach Anspruch 2,
wobei die zweite(n) Rohrdüse(n) jeweils einen Abfluss aufweisen, der in den Innenraum des Reaktors mündet; und
wobei die Organe oder Gewebe jeweils so über die Rohrdüse aufgezogen sind, dass der Abfluss nicht durch das Organ oder Gewebe verdeckt ist.

4. Verfahren nach Anspruch 2, wobei die zweite(n) Rohrdüse(n) jeweils an der dem Zufluss gegenüberliegenden Ende einen weiteren Zufluss aufweisen, durch den ebenfalls von außerhalb des Reaktors Flüssigkeit zuführbar ist, und wobei diese weiteren Zuflüsse in Schritt (1a) berücksichtigt werden.

5. Verfahren nach Anspruch 1, wobei jedes der zu dezellularisierenden Organe oder Gewebe zwischen den Rohrdüsen positioniert ist.

6. Verfahren nach einem der Ansprüche 1 bis 5, wobei die Rohrdüsen aus chemisch stabilen Werkstoffen gefertigt sind und wobei:
mindestens eine Rohrdüse aus einem festen Material gefertigt ist; und/oder
mindestens eine Rohrdüse aus einem elastischen Material gefertigt ist und so konfiguriert ist, dass die mindestens eine aus elastischem Material gefertigte Rohrdüse flexibel durch ein Organ oder Gewebe führbar ist,
wobei vorzugsweise, sofern dieser Anspruch von Anspruch 2 abhängt, die aus elastischem Material gefertigten Rohrdüsen zumindest einem Teil der zweiten Rohrdüsen entsprechen.

7. Verfahren nach einem der Ansprüche 1 bis 6, wobei Schritt (1a) des Pressens und Schritt (1b) des Ansaugens der Reinigungslösung mit einer oder mehrerer Pumpe(n), vorzugsweise Liquidpumpe(n), durchgeführt werden, wobei der Ausgang der Pumpe(n) über ein Schlauchsystem mit den Zuflüssen und der Eingang der Pumpe(n) mittels eines Schlauchs oder Schlauchsystems mit dem Abfluss verbunden ist.

8. Verfahren nach einem der Ansprüche 1 bis 7, wobei das Verfahren mehrmals unterbrochen wird, um die alte Reinigungslösung gegen neue Reinigungslösung auszutauschen.

9. Verfahren nach einem der Ansprüche 1 bis 8, mit den vorbereitenden Schritten:
(9a) Entnahme der Organe oder Gewebe von toten Körpern;
(9b) Säuberung der Organe oder Gewebe, beispielsweise von Fett oder Tunica adventitia;
(9c) Einbau der Organe oder Gewebe in den Reaktor durch Aufziehen der Organe oder Gewebe auf je eine der zweiten Rohrdüsen oder Führen der zweiten Rohrdüsen durch die Organe oder Gewebe;
(9d) Fixierung der Organe oder Gewebe an den Rohrdüsen;
(9e) Herstellen der Reinigungslösung;
(9f) Befüllen des Reaktors mit der Reinigungslösung;
(9g) Anschließen des Reaktors mittels eines geschlossenen Schlauchsystems an die Pumpe;
(9h) Einstellen und Starten der Pumpe;
wobei die Schritte (9a) bis (9h) dem Schritt (1a) gemäß Anspruch 1 vorangehen.

10. Verfahren nach einem der Ansprüche 1 bis 9, mit den weiteren Schritten:
(10a) Beseitigen der an den extrazellulären Matrizen gebundenen Reinigungslösung;
(10b) Entnehmen der dezellularisierten Organe oder Gewebe (extrazellulären Matrizen) aus dem Reaktor;
und den folgenden jeweils optionalen Schritten:
(10c) Messen der Konzentration der verbliebenen Reinigungslösung in den dezellularisierten Organen oder Geweben (extrazellulären Matrizen); und/oder
(10d) Überführen der extrazellulären Matrizen in Reaktionsgefäße und anschließendem Befüllen der Reaktionsgefäße mit phosphatgepufferter Salzlösung (PBS); und/oder
(10e) Sterilisation der extrazellulären Matrizen mit Gammastrahlen; und/oder
(10f) Lagerung der extrazellulären Matrizen bei 1° bis 14° C.

11. Verfahren nach Anspruch 10, wobei Schritt (10a) des Beseitigens der an den extrazellulären Matrizen gebundenen Reinigungslösung die folgenden Schritte aufweist:
(11a) Verbinden des Reaktors, einer ersten Pumpe, einem Reservoir, einem oder mehreren Reinigungsfiltern, die sich im Reservoir befinden und jeweils einen Zulauf sowie ein Überlaufventil oder ein Loch aufweisen, und einer zweiten Pumpe mittels eines Schlauchsystems so, dass:
(i) der Abfluss des Reaktor durch einen ersten Schlauch mit dem Eingang der ersten Pumpe verbunden ist,
(ii) der Ausgang der ersten Pumpe durch einen zweiten Schlauch oder ein weiteres Schlauchsystem mit dem Zufluss/den Zuflüssen des/der Reinigungsfilter(s) verbunden ist,
(iii) das Innere des Reservoirs durch einen dritten Schlauch mit dem Eingang der zweiten Pumpe verbunden ist, und
(iv) der Ausgang der zweiten Pumpe durch einen vierten Schlauch mit dem Zufluss oder den Zuflüssen des Reaktors verbunden ist;
(11b) Befüllen des Reservoirs mit einer Waschflüssigkeit;
(11c) Einstellen der Flussraten an der ersten Pumpe und an der zweiten Pumpe auf einen gleichen Wert;
(11d) Starten der Pumpen.

12. Verfahren nach Anspruch 11, wobei die Reinigungsfilter jeweils aufweisen:
ein Reaktionsgefäß, das mit einem Adsorptionsmittel befüllt ist;
einen Deckel, mit dem das Reaktionsgefäß verschlossen ist,
wobei der Deckel aufweist:
einen Zulauf und
ein Überlaufventil, einen Abfluss oder ein Loch.

13. Verfahren nach Anspruch 12, wobei:
das Adsorptionsmittel Aktivkohle aufweist; und/oder
das Adsorptionsmittel Zeolithe, Kunstharze, Tonmineralien, Aluminiumoxid, Holz, Sepharose und/oder Silikatverbindungen aufweist.

14. Verfahren nach einem der Ansprüche 11 bis 13,
wobei die Waschflüssigkeit im Reservoir und/oder im Reaktor für einen Zeitraum von mehreren Tagen zumindest einmal täglich durch neue Waschflüssigkeit ausgetauscht wird;
wobei die Entnahme der dezellularisierten Organe oder Gewebe aus dem Reaktor gemäß Schritt (10b) nach dem genannten Zeitraum erfolgt.

15. Verfahren nach einem der Ansprüche 10 bis 14, wobei das Messen der Konzentration der verbliebenen Reinigungsflüssigkeit in den extrazellulären Matrizen die folgenden Schritte aufweist:
(15a) Bereitstellen einer Reaktionsflüssigkeit, die Chloroform und Methanol umfasst;
(15b) Geben von Waschflüssigkeit oder homogenisierter Matrix zur Reaktionsflüssigkeit;
(15c) Zugeben einer Methylenblaulösung;
(15d) intensives Mischen;
(15e) Abwarten von mindestens 5 min;
(15f) photometrisches Bestimmen der Absorption der Chloroformphase mit Hilfe einer zuvor erstellten Eichkurve der Konzentration der SDS-Lösung.

16. Reaktor zur Dezellularisierung von Organen oder Geweben, wobei der Reaktor aufweist:
mindestens eine erste, äußere Rohrdüse und mindestens eine zweite, innere Rohrdüse, wobei:
die Rohrdüsen im Innenraum des Reaktors montiert sind;
die zweite, innere Rohrdüse derart konfiguriert ist, dass das Organ oder Gewebe darauf aufziehbar ist und
in die Rohrdüsen von außerhalb des Reaktors durch einen Zufluss
Flüssigkeit zugeführt werden kann zur inneren und äußerlichen Behandlung der zu dezellularisierenden Organe oder Gewebe;
einen Abfluss.

17. Reaktor nach Anspruch 16,
wobei die zweiten Rohrdüsen jeweils so konfiguriert sind, dass ein Organ über die Länge der zweiten Rohrdüse aufziehbar ist.

18. System zur Dezellularisierung von Organen oder Geweben, wobei das System aufweist:
den Reaktor gemäß Anspruch 16 oder 17;
eine Pumpe;
wobei:
der Ausgang der Pumpe über ein Schlauchsystem mit den Zuflüssen der des Reaktors verbunden ist; und
der Eingang der Pumpe mittels eines Schlauchs mit dem Abfluss des Reaktors verbunden ist.

19. System zum Beseitigen von an dezellularisierten Organen oder Geweben gebundener Reinigungslösung, wobei das System aufweist:
den Reaktor gemäß Anspruch 16 oder 17;
eine erste Pumpe, vorzugsweise eine Liquidpumpe;
ein Reservoir;
ein oder mehrere Reinigungsfilter, die sich im Reservoir befinden und jeweils einen Zulauf sowie ein Überlaufventil oder ein Loch aufweisen;
eine zweite Pumpe;
wobei der Reaktor, die erste Pumpe, das Reservoir, der oder die mehreren Reinigungsfilter und die zweite Pumpe mittels eines Schlauchsystems so verbunden sind, dass:
(i) der Abfluss des Reaktor durch einen ersten Schlauch mit dem Eingang der ersten Pumpe verbunden ist,
(ii) der Ausgang der ersten Pumpe durch einen zweiten Schlauch oder ein weiteres Schlauchsystem mit dem Zufluss/den Zuflüssen des/der Reinigungsfilter(s) verbunden ist,
(iii) das Innere des Reservoirs durch einen dritten Schlauch mit dem Eingang der zweiten Pumpe verbunden ist, und
(iv) der Ausgang der zweiten Pumpe durch einen vierten Schlauch mit dem Zufluss oder den Zuflüssen des Reaktors verbunden ist;
und wobei:
die erste Pumpe ein System von mehreren parallel zusammengeschlossenen Pumpen aufweisen kann;
die zweite Pumpe ein System von mehreren parallel zusammengeschlossenen Pumpen aufweisen kann.

20. System nach Anspruch 19, wobei die Reinigungsfilter jeweils aufweisen:
ein Reaktionsgefäß, das mit einem Adsorptionsmittel befüllt ist;
einen Deckel, mit dem das Reaktionsgefäß verschlossen ist,
wobei der Deckel aufweist:
einen Zulauf und
ein Überlaufventil, einen Abfluss oder ein Loch;
und wobei:
das Adsorptionsmittel Aktivkohle aufweist und/oder
das Adsorptionsmittel Zeolithe, Kunstharze, Tonmineralien, Aluminiumoxid, Holz, Sepharose und/oder Silikatverbindungen aufweist.

## Claims

1. Method for decellularising organs and tissues in a reactor, wherein the reactor comprises:
at least one first external tubular nozzle and at least one second internal tubular nozzle, wherein:
the tubular nozzles are mounted in the interior of the reactor; and fluid can be fed into the tubular nozzles from outside of the reactor through an inlet;
an outlet;
wherein each of the organs or tissues to be decellularised is positioned in the interior of the reactor and is mounted on a second internal tubular nozzle;
wherein a cleaning solution is present in the reactor; and
wherein the method comprises the following steps:
(1a) pressing a cleaning solution with pressure through the respective inlets into first and second tubular nozzles for the internal and external treatment of the organs and tissues to be decellularised;
(1b) aspirating cleaning fluid from the reactor through the outlet;
wherein:
the pressure in step (1a) is selected such that, in the reactor, strong laminar and/or turbulent flows and eddies occur in the cleaning solution; and steps (1a) and (1b) are preferably carried out simultaneously and over a predetermined period of time.

2. Method of claim 1,
wherein the second tubular nozzles are each adapted such that an organ can be mounted over the length of the second tubular nozzle; and
wherein each of the organs and tissues to be decellularised is mounted over the length of one of the second tubular nozzles.

3. Method of claim 2,
wherein the second tubular nozzle(s) each comprise(s) an outlet which opens into the interior of the reactor; and
wherein the organs or tissues are mounted over the tubular nozzles such that the outlet is not covered by the organ or the tissue.

4. Method of claim 2, wherein the second tubular nozzle(s) each comprise(s) a further inlet at the end opposite the inlet, through which fluid can also be fed from outside of the reactor, and wherein said further inlets are used in step (1a).

5. Method of claim 1, wherein each of the organs and tissues to be decellularised is positioned between the tubular nozzles.

6. Method of any one of claims 1 to 5, wherein the tubular nozzles are made of chemically stable materials and wherein:
at least one tubular nozzle is made of a rigid material; and/or
at least one tubular nozzle is made of an elastic material and is adapted such that the at least one tubular nozzle made of elastic material can be conducted through an organ or tissue in a flexible way,
wherein it is preferred that, if this claim is dependent on claim 2, the tubular nozzles made of elastic material correspond to at least part of the second tubular nozzles.

7. Method of any one of claims 1 to 6, wherein step (1a) of pressing and step (1b) of aspirating the cleaning solution are carried out with one or more pump(s), preferably liquid pump(s), wherein the outlet of the pump(s) is connected with the inlets via a tube system and the inlet of the pump(s) is connected with the outlet via a tube or tube system.

8. Method of any one of claims 1 to 7, wherein the method includes multiple interruptions allowing the exchange of the old cleaning solution for a new cleaning solution.

9. Method of any one of claims 1 to 8 with the preparatory steps:
(9a) removing the organs or tissues from dead bodies;
(9b) cleaning the organs or tissues, for example, cleaning off fat or tunica adventiva;
(9c) positioning the organs or tissues in the reactor by mounting the organs or tissues on one of the second tubular nozzles each or conducting the second tubular nozzles through the organs and tissues;
(9d) fixing the organs or tissues at the tubular nozzles;
(9e) preparing the cleaning solution;
(9f) filling the reactor with the cleaning solution;
(9g) connecting the reactor with the pump by means of a closed tube system;
(9h) setting and starting the pump;
with steps (9a) to (9h) preceding step (1a) of claim 1.

10. Method of any one of claims 1 to 9 with the further steps:
(10a) removing the cleaning solution bound to the extracellular matrices;
(10b) taking the decellularised organs or tissues (extracellular matrices) from the reactor;
and the following optional steps:
(10c) measuring the concentration of the remaining cleaning solution in the decellularised organs or tissues (extracellular matrices); and/or
(10d) transferring the extracellular matrices into reaction vessels and subsequently filling the reaction vessels with phosphate-buffered saline (PBS); and/or
(10e) sterilising the extracellular matrices by gamma radiation; and/or
(10f) storing the extracellular matrices at 1°C to 14°C.

11. Method of claim 10, wherein step (10a) of removing the cleaning solution bound to the extracellular matrices comprises the following steps:
(11a) connecting the reactor, a first pump, a reservoir, one or more purifying filter(s) which are located in the reservoir and each of which has an inlet and an overflow valve or an opening and a second pump by means of a tube system so that:
(i) the outlet of the reactor is connected with the inlet of the first pump via a first tube,
(ii) the outlet of the first pump is connected with the inlet(s) of the purifying filter(s) via a second tube or a further tube system,
(iii) the interior of the reservoir is connected with the inlet of the second pump via a third tube,
(iv) the outlet of the second pump is connected with the inlet or the inlets of the reactor via a fourth tube;
(11b) filling the reservoir with a washing liquid;
(11c) setting the flow rates of the first pump and of the second pump to an identical value;
(11d) starting the pumps.

12. Method of claim 11, wherein each of the purifying filters comprises:
a reaction vessel filled with an adsorbant;
a lid by means of which the reaction vessel is closed,
wherein the lid comprises:
an inlet and
an overflow valve, an outlet or an opening.

13. Method of claim 12, wherein:
the adsorbant comprises activated carbon; and/or
the adsorbant comprises zeolites, artificial resins, clay minerals, aluminium oxide, wood, sepharose and/or silicate compounds.

14. Method of any one of claims 11 to 13,
wherein the washing liquid in the reservoir and/or in the reactor is exchanged for new washing liquid at least once a day over a period of several days;
wherein the taking of the decellularised organs and tissues from the reactor according to step (10b) is carried out after said period of time.

15. Method of any one of claims 10 to 14, wherein measuring the concentration of the remaining cleaning fluid in the extracellular matrices comprises the following steps:
(15a) providing a reaction liquid which comprises chloroform and methanol;
(15b) adding washing liquid or homogenized matrix to the reaction liquid;
(15c) adding a methylene blue solution;
(15d) intensive mixing;
(15e) leaving to rest for at least 5 min;
(15f) photometric determination of the absorption of the chloroform phase using a calibration curve of the concentration of the SDS solution which was established before.

16. Reactor for decellularising organs or tissues, wherein the reactor comprises:
at least one first, external tubular nozzle and at least one second, internal tubular nozzle,
wherein:
the tubular nozzles are mounted in the interior of the reactor;
the second, internal tubular nozzle is adapted such that the organ or the tissue can be mounted thereon and
liquid for internal and external treatment of the organs and tissues to be decellularised can be fed from outside of the reactor through an inlet into the tubular nozzles;
an outlet.

17. Reactor of claim 16,
wherein the second tubular nozzles are each adapted such that an organ can be mounted over the length of the second tubular nozzle.

18. System for decellularising organs or tissues, wherein the system comprises:
the reactor of claim 16 or 17;
a pump;
wherein:
the outlet of the pump is connected with the inlets of the reactor via a tube system; and
the inlet of the pump is connected with the outlet of the reactor via a tube.

19. System for removing cleaning fluid bound on decellularised organs or tissues; wherein the system comprises:
the reactor of claim 16 or 17;
a first pump, preferably a liquid pump;
a reservoir;
one or more purifying filter(s) located in the reservoir and each having an inlet and an overflow valve or an opening;
a second pump;
wherein the reactor, the first pump, the reservoir, said one or more purifying filter(s) and the second pump are connected via a tube system such that:
(i) the outlet of the reactor is connected via a first tube with the inlet of the first pump,
(ii) the outlet of the first pump is connected with the inlet(s) of the purifying filter(s) via a second tube or a further tube system,
(iii) the interior of the reservoir is connected with the inlet of the second pump via a third tube, and
(iv) the outlet of the second pump is connected with the inlet or the inlets of the reactor via a fourth tube;
and wherein:
the first pump may comprise a system of multiple pumps connected in parallel;
the second pump may comprise a system of several pumps connected in parallel.

20. System of claim 19, wherein the purifying filters each comprise:
a reaction vessel which is filled with an adsorbant,
a lid with which the reaction vessel is closed,
wherein the lid comprises:
an inlet and
an overflow valve, an outlet or an opening;
and wherein:
the adsorbant comprises activated carbon and/or
the adsorbant comprises zeolites, artificial resins, clay minerals, aluminium oxide, wood, sepharose and/or silicate compounds.

## Revendications

1. Procédé de décellularisation d'organes ou de tissus dans un réacteur, le réacteur présentant :
au moins une première buse tubulaire externe et au moins une deuxième buse tubulaire interne, dans lequel :
les buses tubulaires sont montées à l'intérieur du réacteur ; et
dans les buses tubulaires de l'extérieur du réacteur, un liquide peut être acheminé par une arrivée ;
une évacuation ;
dans lequel chacun des organes ou tissus à décellulariser est positionné à l'intérieur du réacteur et est appliqué sur une deuxième buse tubulaire interne ;
dans lequel se trouve une solution de nettoyage dans le réacteur ; et
le procédé présentant les étapes suivantes :
(1a) le passage d'une solution de nettoyage par une pression dans les arrivées respectives dans les première et deuxième buses tubulaires pour le traitement interne et externe des organes ou tissus à décellulariser ;
(1b) l'aspiration du liquide de nettoyage par l'évacuation hors du réacteur ;
dans lequel
la pression dans l'étape (1a) est choisie de telle sorte que dans le réacteur, de forts courants laminaires et/ou turbulents et des tourbillons se produisent dans la solution de nettoyage ; et
les étapes (1a) et (1b) sont réalisées de préférence de façon concomitante et pendant une durée d'un temps prédéterminé.

2. Procédé selon la revendication 1,
dans lequel les deuxièmes buses tubulaires sont configurées respectivement de telle sorte qu'un organe peut être appliqué sur la longueur de la deuxième buse tubulaire ; et
dans lequel chacun des organes ou tissus à décellulariser est appliqué le long de l'une respective des deuxièmes buses tubulaires.

3. Procédé selon la revendication 2,
dans lequel la/les deuxième(s) buse(s) tubulaire(s) présentent respectivement une évacuation qui débouche dans l'intérieur du réacteur ; et
dans lequel les organes ou tissus sont appliqués respectivement sur la buse tubulaire de telle sorte que l'évacuation ne soit pas couverte par l'organe ou le tissu.

4. Procédé selon la revendication 2, dans lequel la/les deuxième(s) buse(s) tubulaire(s) présentent respectivement sur l'extrémité opposée à l'arrivée, une autre arrivée par laquelle également de l'extérieur du réacteur, un liquide peut être acheminé, et dans lequel ces autres arrivées sont prises en compte dans l'étape (1a).

5. Procédé selon la revendication 1, dans lequel chacun des organes ou tissus à décellulariser est placé entre les buses tubulaires.

6. Procédé selon l'une des revendications 1 à 5, dans lequel les buses tubulaires sont fabriquées en matériaux chimiquement stables et dans lequel :
au moins une buse tubulaire est fabriquée en un matériau rigide ; et/ou au moins une buse tubulaire est fabriquée en un matériau élastique et est configurée de telle sorte que ladite buse tubulaire fabriquée en matériau élastique puisse être guidée de façon souple dans un organe ou un tissu,
dans lequel de préférence, dans la mesure où cette revendication dépend de la revendication 2, les buses tubulaires fabriquées en matériau élastique correspondent au moins à une partie des deuxièmes buses tubulaires.

7. Procédé selon l'une des revendications 1 à 6, dans lequel l'étape (1a) du passage et l'étape (1b) de l'aspiration de la solution de nettoyage sont réalisées avec une ou plusieurs pompe(s), de préférence une/des pompes à liquide, dans lequel la sortie de la/des pompe(s) est reliée par un système de tuyaux aux arrivées et l'entrée de la/des pompe(s) est reliée au moyen d'un tuyau ou d'un système de tuyaux à l'évacuation.

8. Procédé selon l'une des revendications 1 à 7, dans lequel le procédé est interrompu plusieurs fois pour échanger l'ancienne solution de nettoyage contre une nouvelle solution de nettoyage.

9. Procédé selon l'une des revendications 1 à 8, comportant les étapes de préparation suivantes :
(9a) prélèvement des organes ou tissus de corps morts ;
(9b) nettoyage des organes ou tissus, par exemple, des graisses ou de l'adventice ;
(9c) mise en place des organes ou tissus dans le réacteur par application des organes ou tissus sur l'une respective des deuxièmes buses tubulaires ou guidage des deuxièmes buses tubulaires dans les organes ou tissus ;
(9d) fixation des organes ou tissus sur les buses tubulaires ;
(9e) production de la solution nettoyante ;
(9f) remplissage du réacteur avec la solution nettoyante ;
(9g) raccord du réacteur au moyen d'un système de tuyaux fermé à la pompe ;
(9h) réglage et mise en route de la pompe ;
dans lequel les étapes (9a) à (9h) précèdent l'étape (1a) selon la revendication 1.

10. Procédé selon l'une des revendications 1 à 9, comprenant les autres étapes suivantes :
(10a) élimination de la solution de nettoyage fixée aux matrices extracellulaires ;
(10b) prélèvement des organes ou tissus décellularisés (matrices extracellulaires) du réacteur ;
et les étapes optionnelles respectives suivantes :
(10c) mesure de la concentration de la solution de nettoyage restante dans les organes ou tissus décellularisés (matrices extracellulaires) ; et/ou (10d) transfert des matrices extracellulaires dans des récipients réactionnels et ensuite, remplissage des récipients réactionnels avec une solution physiologique tamponnée au phosphate (PBS) ; et/ou
(10e) stérilisation des matrices extracellulaires avec des rayons gamma ; et/ou
(10f) stockage des matrices extracellulaires de 1°C à 14°C.

11. Procédé selon la revendication 10, dans lequel l'étape (10a) d'élimination de la solution de nettoyage fixée aux matrices extracellulaires présente les étapes suivantes :
(11a) liaison du réacteur, à une première pompe, un réservoir, un ou plusieurs filtres de nettoyage qui se trouvent dans le réservoir et présentent respectivement une amenée et une valve anti-débordement ou un trou et à une deuxième pompe au moyen d'un système de tuyaux, de sorte que :
(i) l'évacuation du réacteur soit reliée par un premier tuyau à l'arrivée de la première pompe,
(ii) la sortie de la première pompe soit reliée par un deuxième tuyau ou un autre système de tuyaux à l'arrivée/ aux arrivées du/des filtre(s) de nettoyage,
(iii) l'intérieur du réservoir soit relié par un troisième tuyau à l'entrée de la deuxième pompe, et
(iv) la sortie de la deuxième pompe soit reliée par un quatrième tuyau à l'arrivée ou aux arrivées du réacteur ;
(11b) remplissage du réservoir avec un liquide de lavage ;
(11c) réglage des débits de la première pompe et de la deuxième pompe à une valeur identique ;
(11d) mise en route des pompes.

12. Procédé selon la revendication 11, dans lequel les filtres de nettoyage présentent respectivement :
un récipient réactionnel qui est rempli d'un agent d'adsorption ;
un couvercle avec lequel le récipient réactionnel est fermé,
dans lequel le couvercle présente :
une amenée et
une valve anti-débordement, une évacuation ou un trou.

13. Procédé selon la revendication 12, dans lequel :
l'agent d'adsorption présente des charbons actifs ; et/ou
l'agent d'adsorption présente des zéolithes, des résines synthétiques, des minéraux argileux, de l'oxyde d'aluminium, du bois, du Sépharose et/ou des composés de silicate.

14. Procédé selon l'une des revendications 11 à 13,
dans lequel le liquide de lavage dans le réservoir et/ou dans le réacteur pendant une durée de plusieurs jours est échangé au moins une fois par jour contre un nouveau liquide de lavage ;
dans lequel le prélèvement des organes ou tissus décellularisés hors du réacteur selon l'étape (10b) s'effectue après la durée indiquée.

15. Procédé selon l'une des revendications 10 à 14, dans lequel la mesure de la concentration du liquide de nettoyage restant dans les matrices extracellulaires présente les étapes suivantes :
(15a) mise à disposition d'un liquide réactionnel qui comprend du chloroforme et du méthanol ;
(15b) addition de liquide de lavage ou d'une matrice homogénéisée au liquide réactionnel ;
(15c) addition d'une solution de bleu de méthylène ;
(15d) mélange intensif ;
(15e) attente pendant au moins 5 min ;
(15f) détermination photométrique de l'absorption de la phase de chloroforme à l'aide d'une courbe d'étalonnage produite précédemment de la concentration de la solution de SDS.

16. Réacteur pour la décellularisation d'organes ou de tissus, le réacteur présentant :
au moins une première buse tubulaire externe et au moins une deuxième buse tubulaire interne, dans lequel :
les buses tubulaires sont montées à l'intérieur du réacteur ;
la deuxième buse interne est configurée de telle sorte que l'organe ou le tissu puisse être appliqué sur celle-ci et
dans les buses tubulaires, de l'extérieur du réacteur, un liquide peut être acheminé par une arrivée pour le traitement interne et externe des organes ou tissus à décellulariser ;
une évacuation.

17. Réacteur selon la revendication 16,
dans lequel les deuxièmes buses tubulaires sont respectivement configurées de telle sorte qu'un organe puisse être appliqué sur la longueur de la deuxième buse tubulaire.

18. Système de décellularisation d'organes ou tissus, le système présentant :
le réacteur selon la revendication 16 ou 17 ;
une pompe ;
dans lequel :
la sortie de la pompe est reliée par un système de tuyaux aux arrivées de celle du réacteur ; et
l'entrée de la pompe est reliée au moyen d'un tuyau à l'évacuation du réacteur.

19. Système d'élimination de solution de nettoyage fixée aux organes ou tissus décellularisés, le système comprenant :
le réacteur selon la revendication 16 ou 17 ;
une première pompe, de préférence une pompe à liquide ;
un réservoir ;
un ou plusieurs filtres de nettoyage qui se trouvent dans le réservoir et présentent respectivement une amenée et une valve anti-débordement ou un trou ;
une deuxième pompe ;
dans lequel le réacteur, la première pompe, le réservoir, le ou les multiples filtres de nettoyage et la deuxième pompe sont reliés au moyen d'un système de tuyaux, de sorte que :
(i) l'évacuation du réacteur soit reliée par un premier tuyau à l'arrivée de la première pompe,
(ii) la sortie de la première pompe soit reliée par un deuxième tuyau ou un autre système de tuyaux à l'arrivée/ aux arrivées du/des filtre(s) de nettoyage,
(iii) l'intérieur du réservoir soit relié par un troisième tuyau à l'entrée de la deuxième pompe, et
(iv) la sortie de la deuxième pompe soit reliée par un quatrième tuyau à l'arrivée ou aux arrivées du réacteur ;
et dans lequel :
la première pompe peut présenter un système de plusieurs pompes reliées en parallèle ;
la deuxième pompe peut présenter un système de plusieurs pompes reliées en parallèle.

20. Système selon la revendication 19, dans lequel les filtres de nettoyage présentent respectivement :
un récipient réactionnel qui est rempli d'un agent d'adsorption ;
un couvercle avec lequel le récipient réactionnel est fermé,
dans lequel le couvercle présente :
une amenée et
une valve anti-débordement, une évacuation ou un trou ;
et dans lequel
l'agent d'adsorption présente des charbons actifs et/ou
l'agent d'adsorption présente des zéolithes, des résines synthétiques, des minéraux argileux, de l'oxyde d'aluminium, du bois, du Sépharose et/ou des composés de silicate.
